Europäisches Patentamt

European Patent Office

Office européen des brevets

(19)

(11) Publication number: **0 118 977**
**A1**

(12) **EUROPEAN PATENT APPLICATION**

(21) Application number: **84300439.1**

(22) Date of filing: **25.01.84**

(51) Int. Cl.³: **C 12 N 15/00**
**C 12 P 21/00, C 07 C 103/52**
**C 12 N 5/00, A 61 K 37/02**
**C 12 P 19/30**

(30) Priority: **08.02.83 GB 8303383**
**10.06.83 GB 8315981**

(43) Date of publication of application:
**19.09.84 Bulletin 84/38**

(84) Designated Contracting States:
**AT BE CH DE FR GB IT LI LU NL SE**

(71) Applicant: **BIOGEN N.V.**
**15 Pietermaai**
**Willemstad Curacao, Netherlands Antilles(NL)**

(72) Inventor: **Fiers, Walter Charles**
**Beukendreef 3**
**B-9120 Destelbergen(BE)**

(72) Inventor: **Devos, Rene Robert**
**Edith Cavellstreet 56**
**B-8400 Oostende(BE)**

(74) Representative: **Hartley, David et al,**
**c/o Withers & Rogers 4 Dyer's Buildings Holborn**
**London, EC1N 2JT(GB)**

(54) DNA sequences, recombinant DNA molecules and processes for producing human interleukin two-like polypeptides.

(57) DNA sequences, recombinant DNA molecules and hosts transformed with them which produce polypeptides displaying a biological or immunological activity of human interleukin 2, the genes coding for these polypeptides and methods of making and using these DNA sequences, molecules, hosts, genes and polypeptides. The DNA sequences are characterized in that they code for a polypeptide displaying a biological or immunological activity of human interleukin 2. In appropriate hosts these DNA sequences and recombinant DNA molecules permit the production and identifiation of genes and polypeptides displaying a biological or immunological activity of human interleukin 2 and their use in immunotherapeutic agents and methods and in the establishment and longterm proliferation of T-cells.

DNA SEQUENCES, RECOMBINANT DNA MOLECULES
AND PROCESSES FOR PRODUCING HUMAN
INTERLEUKIN TWO-LIKE POLYPEPTIDES

## TECHNICAL FIELD OF INVENTION

This invention relates to DNA sequences, recombinant DNA molecules and processes for producing human interleukin 2-like polypeptides.  More particularly, the invention relates to DNA sequences and their expression in appropriate host organisms.  The recombinant DNA molecules described herein are characterized by DNA sequences that code for polypeptides which have an immunological or biological activity of human interleukin 2.  As will be appreciated from the disclosure to follow, the DNA sequences, recombinant DNA molecules and processes of this invention may be used in the production of polypeptides useful in a variety of medicinal and other useful commercial applications.

## BACKGROUND ART

Interleukin 2 ("IL2") is a lymphokine. Lymphokines are products synthesized by lymphocytes on stimulation by mitogens.  They are soluble immune response regulatory compounds.  A number of lymphokines are important for their medicinal and laboratory applications.  For example, immune interferon (IFN-$\gamma$) is a lymphokine having antiviral, anticancer and immuno-modulation activity [e.g., R. Devos et al.,

Nucleic Acids Research, 10, pp. 2487-501 (1982) and references cited therein].

Interleukin 2 is another lymphokine having important and useful biological properties [Gillis et al., Immunological Rev., 63, pp. 166-209 (1982)]. For example IL2 (a) stimulates the long-term proliferation of antigen-specific effector T-cells in vitro; (b) enhances thymocyte mitogenesis; and (c) induces cytotoxic T-cell reactivity and plaque-forming responses in cultures of nude mouse spleen cells [Gillis & Watson, J. Exp. Med., 152, pp. 1709-19 (1980); J.J. Farrar et al., Immunological Rev., 63, pp. 129-65 (1982)].

As a result of its biological properties, interleukin 2 may be used to stimulate the recruitment, for example, by thymic stimulation, of human leukocyte antigen restricted tumor-specific cytotoxic cells in cancer patients who have impaired immune systems. Treatment with IL2 will thus enable these patients better to resist a variety of bacterial and other infections. Such infections often complicate cancer therapy, particularly radiation and chemotherapy.

In addition, IL2's ability to stimulate the immune system may make it useful in the treatment of diseases which are related to the malfunctioning of the immune system, e.g., AIDS, lupus, multiple sclerosis and rheumatoid arthritis. Published pre-clinical studies have demonstrated a relationship between these diseases and abnormalities in T-lymphocytes or the production of abnormal antibodies. For example, in vitro studies suggest that at least some functions of the immune system may be able to be restored to certain AIDS patients by the administration of IL2. Moreover, in vitro studies have indicated that low levels of IL2 are associated with the production of abnormal antibodies related to some of these diseases of the immune system.

In addition, based on published studies, it is believed that IL2 may be useful in treating a broad range of cancers. Furthermore, it may be particularly useful as adjunctive therapy to treat solid tumors, e.g., lung, colon-rectum and breast cancers. IL2 may also be useful in treating herpes genitalis and other viral diseases, either alone or in adjunctive therapy.

It has also been shown that the anticancer activity of interferon is enhanced, and may even require, IL2, because IL2 stimulates the full activation of the "natural killer" cells which are the presumed major source of interferon's anticancer activity [C.S. Henney, Nature, 291, pp. 335-38 (1981)]. IL2 may also be useful in avoiding the deficiency of appropriate suppressor cells that characterizes several immune diseases, such as multiple sclerosis.

Finally IL2 is widely used for the establishment and long-term proliferation of cultures of T-cells for diagnostic and therapeutic purposes. For example, cancer cells are now employed to permit selection of homologous cytotoxic T-cells. These T-cells are then cultured in vitro and reinjected into the patient to effect tumor regression.

On a biochemical level IL2 was believed to have a molecular weight of about 15000 daltons and to be glycosylated. Assuming it were glycosylated, it should then have between 100 and 130 amino acids. IL2 behaves somewhat heterogeneously on columns. On SDS gels, two bands (12000 and 13000 daltons), possibly subunits, are observed [J.W. Mier and R.C. Gallo, Proc. Natl. Acad. Sci. USA, 77, pp. 6134-38 (1980)]. IL2 may also be more than a single compound. It, like leukocyte interferon (IFN-α), may be a family of products displaying varying levels of immunotherapeutic activity. IL2 may also be polymorphic. For
example, cells of particular individuals may produce
IL2 species within the more general IL2 class, which
are physiologically similar but structurally slightly
different from the prototype of the class of which
it is a part.

One of the lymphokines -- IFN-γ -- has
been produced in large amounts by the application of
genetic engineering [R. Devos et al., supra]. IL2
has not been produced in this manner.

However, because of the numerous important
immunotherapeutic activities of IL2 and because of
its required use in the establishment and long-term
proliferation of T-cells for diagnostic and therapeutic purposes, various methods and means have been
investigated and considered for the large scale production of IL2. None has been successful.

## DISCLOSURE OF THE INVENTION

The present invention solves the problems
referred to by locating and identifying DNA sequences
that code for IL2 and transforming appropriate hosts
with those sequences thereby providing DNA sequences,
recombinant DNA molecules and methods for the use of
those sequences and molecules in the production of
polypeptides displaying an immunological or biological
activity of human interleukin 2.

By virtue of this invention, it is possible
to obtain polypeptides displaying an immunological
or biological activity of IL2 for use in immunotherapeutic agents and methods and in the establishment
and long-term proliferation of T-cells. This inven-
tion allows the production of these polypeptides in
amounts and by methods hitherto not available.

As will be appreciated from the disclosure
to follow, the DNA sequences and recombinant DNA molecules of the invention are capable of directing the production, in an appropriate host, of polypeptides displaying an immunological or biological activity of IL2. Replication of these DNA sequences and recombinant DNA molecules in appropriate hosts also permits the production in large quantities of genes coding for these polypeptides. The molecular structure and properties of these polypeptides and genes may thus be readily determined. The polypeptides and genes are useful, either as produced in the host or after appropriate derivatization or modification, in compositions and methods for detecting and improving the production of these products themselves and for use in immunotherapeutic agents and methods and in compositions and methods for the establishment and long-term proliferation of T-cells.

It will be appreciated from the foregoing that a basic aspect of this invention is the provision of a DNA sequence, which is characterized in that it codes for a polypeptide displaying an immunological or biological activity of IL2, or at least allows the selection of such sequences from a collection of DNA sequences, and which is selected from the group consisting of the DNA inserts hIL2-0, hIL2-1, DNA sequences which hybridize to any of the foregoing DNA inserts and which encode IL2, and DNA sequences which on expression code for a polypeptide coded for on expression by any of the foregoing DNA sequences. The sequences of this invention are further characterized in that they permit the production of IL2 and IL2-like polypeptides in hosts.

## BRIEF DESCRIPTION OF THE DRAWINGS

Figure 1 is a graphical representation of the IFN encoding activity of a series of sucrose-gradient RNA fractions prepared in accordance with this invention.

Figure 2 is a pictorial representation of pSV529 and that vector having an IL2-related DNA insert inserted into it at the BamHI site.

Figure 3 displays a restriction map of an IL2-related DNA sequence of this invention and the sequencing strategy used in determining its DNA sequence.

Figure 4 displays the nucleotide sequence of an IL2-related DNA sequence of this invention.

Figure 5 displays in schematic outline the preparation of various recombinant DNA molecules of this invention that may be employed to transform appropriate hosts which when cultured produce the IL2-like products of this invention.

Figure 6 displays the partial restriction map of plaque·λCH4A-ghIL2-1 which contains the total hIL2 gene.

## BEST MODE OF CARRYING OUT THE INVENTION

In order that the invention herein described may be more fully understood, the following detailed description is set forth.

In the description the following terms are employed:

Nucleotide--A monomeric unit of DNA or RNA consisting of a sugar moiety (pentose), a phosphate, and a nitrogenous heterocyclic base. The base is linked to the sugar moiety via the glycosidic carbon (1' carbon of the pentose) and that combination of base and sugar is called a nucleoside. The base characterizes the nucleotide. The four DNA bases are adenine ("A"), guanine ("G"), cytosine ("C"), and thymine ("T"). The four RNA bases are A, G, C, and uracil ("U").

DNA Sequence--A linear array of nucleotides connected one to the other by phosphodiester bonds between the 3' and 5' carbons of adjacent pentoses.

Codon--A DNA sequence of three nucleotides (a triplet) which encodes through mRNA an amino acid, a translation start signal or a translation termination signal. For example, the nucleotide triplets TTA, TTG, CTT, CTC, CTA and CTG encode for the amino acid leucine ("Leu"), TAG, TAA and TGA are translation stop signals and ATG is a translation start signal.

Reading Frame--The grouping of codons during the translation of mRNA into amino acid sequences. During translation the proper reading frame must be maintained. For example, the DNA sequence GCTGGTTGTAAG may be expressed in three reading frames or phases, each of which affords a different amino acid sequence:

GCT GGT TGT AAG--Ala-Gly-Cys-Lys
G CTG GTT GTA AG--Leu-Val-Val
GC TGG TTG TAA G--Trp-Leu-(STOP)

Polypeptide--A linear array of amino acids connected one to the other by peptide bonds between the α-amino and carboxy groups of adjacent amino acids.

Genome--The entire DNA of a cell or a virus. It includes inter alia the structural gene coding for the polypeptides of the substance, as well as operator, promoter and ribosome binding and interaction sequences, including sequences such as the Shine-Dalgarno sequences.

Gene--A DNA sequence which encodes through its template or messenger RNA ("mRNA") a sequence of amino acids characteristic of a specific polypeptide.

Transcription--The process of producing mRNA from a gene or DNA sequence.

Translation--The process of producing a polypeptide from mRNA.

Expression--The process undergone by a gene or DNA sequence to produce a polypeptide. It is a combination of transcription and translation.

Plasmid--A nonchromosomal double-stranded DNA sequence comprising an intact "replicon" such that the plasmid is replicated in a host cell. When the plasmid is placed within a unicellular organism, the characteristics of that organism may be changed or transformed as a result of the DNA of the plasmid. For example, a plasmid carrying the gene for tetracycline resistance (Tet$^R$) transforms a cell previously sensitive to tetracycline into one which is resistant to it. A cell transformed by a plasmid is called a "transformant".

Phage or Bacteriophage--Bacterial virus many of which consist of DNA sequences encapsidated in a protein envelope or coat ("capsid").

Cloning Vehicle--A plasmid, phage DNA or other DNA sequence which is able to replicate in a host cell, characterized by one or a small number of endonuclease recognition sites at which such DNA sequences may be cut in a determinable fashion without attendant loss of an essential biological function of the DNA, e.g., replication, production of coat proteins or loss of promoter or binding sites, and which contain a marker suitable for use in the identification of transformed cells, e.g., tetracycline resistance or ampicillin resistance. A cloning vehicle is often called a vector.

Cloning--The process of obtaining a population of organisms or DNA sequences derived from one such organism or sequence by asexual reproduction.

Recombinant DNA Molecule or Hybrid DNA--A molecule consisting of segments of DNA from different genomes which have been joined end-to-end outside of living cells and able to be maintained in living cells.

Expression Control Sequence--A sequence of nucleotides that controls and regulates expression of genes when operatively linked to those genes. They include the lac system, major operator and promoter regions of phage λ, the control region of fd coat protein and other sequences known to control the expression of genes of prokaryotic or eukaryotic cells and their viruses or combinations thereof.

IL2-like--A polypeptide displaying a biological or immunological activity of IL2. This polypeptide may include amino acids in addition to those of native IL2 or it may not include all of the amino acids of native IL2.

### BIOLOGICAL ASSAY FOR INTERLEUKIN 2

(a) Principle

Interleukin 2 (IL2) or T-cell growth factor is a soluble compound that permits the long-term culture of cloned derivatives of mitogen or antigen-stimulated T-cells [D.A. Morgan et al., Science 193, pp. 1007-08 (1976)]. The growth and proliferation of these cells depends on the presence of IL2 in the medium in a dose-dependent manner. Therefore, a measurement of the amount of proliferation (i.e., by incorporation of tritiated thymidine) is an indication of the IL2 concentration in the medium.

(b) Target Cells

·We isolated mononuclear cells from human peripheral·blood by means of "lymphoprep" (Pharmacia), washed the cells (at the interphase) twice and cultured them at a concentration of $5 \times 10^5$ cells/ml in RPMI 1640 medium, supplemented with 1% L-glutamine, antibiotics (see below) and 10% fetal calf serum. We then stimulated the cells with phytohemagglutinin (PHA) (Wellcome) to 10 µg/ml for 20 h at 37°C, collected and intensively washed them and cultured them for another 3 days in medium without PHA. On the fourth day, we supplemented the cells with 5% of a partially purified human IL2 preparation (a 50-80% $(NH_4)_2SO_4$ precipitate derived from the medium of PHA-stimulated splenocytes, redissolved in 1/20 of the original volume and exhaustively dialyzed against phosphate buffered saline [Morgan et al., supra]). We then split the cells every three to four days at a ratio of 1:2.

When the cultures were approximately 2 to 3 weeks old, we washed living cells from them and cultured the cells in IL2-free medium with 40% fetal calf serum and 10% dimethylsulfoxide to a final concentration of $2x10^7$ cells/ml. Immediately thereafter, we froze the cells in 1 ml glass vials, using a controlled rate freezer (Cryoson) and cooling rates of 1°C/min to -45°C and then quickly to -80°C. We stored the target cells in liquid nitrogen [Gramatzki et al., J. Immun. Methods, 53, pp. 209-20 (1982)]. Immediately before using the cells, we thawed them rapidly at 37°C, mixed them stepwise with medium and washed them. We examined the viability of our prepared cells by Tyrol blue exclusion.

(c) Microassay

We performed our IL2 microassay substantially as described by Gillis et al., J. Immun., 120, pp. 2027-32 (1978). We first serially diluted (1:2) 100 µl IL2 samples into 96-well microtiter plates (Falcon) (in RPMI 1640, 10% fetal calf serum, 1% L-glutamine and antibiotics, see below). We then suspended the target cells at $2x10^5$ cells/ml and added 100 µl of cells to the wells to a final concentration of about $2x10^4$ cells/well. We incubated the microtiter plates at 37°C for 24 h in a humidified atmosphere of 5% $CO_2$ in air, added 0.5 µ Ci $^3$H-labelled thymidine (Amersham 20-30 Ci/mmole) to each microplate well, and cultured the cells for an additional 5 h. We then harvested the cultures onto glass filter strips (with a cell-harvester; type MASH II) and determined the amount of $^3$H-thymidine incorporation with a liquid scintillation counter.

### (d) Quantification

We determined the relative activity of each test sample using a human IL2 standard sample. The standard (an arbitrarily chosen human IL2-containing supernatant) was defined as containing 100 Units/ ml. It induces maximal proliferation up to dilutions of 1:4 to 1:8 as measured by $^3$H-thymidine uptake. We usually observed 30% of the maximal activity of this sample in the center of the linearly descending part of the standard curve.

We also tested each sample at serial dilutions and calculated their titer by a graphical regression analysis. We then transformed the activity of each sample into units using the formula [Stadler et al., J. Immun., 128, pp. 1620-24 (1982)]:

$$\frac{\text{Reciprocal titer at 30\% of maximum cpm of standard of test sample}}{\text{Reciprocal titer at 30\% of maximum cpm of standard}} = \text{units x 100}$$

### PREPARATION OF POLY(A) RNA CONTAINING HUMAN INTERLEUKIN 2 mRNA (hIL2-mRNA)

The RNA used in the processes of this invention was extracted from mitogen-stimulated human splenocytes. Splenocytes offered to us the advantage that they provided a large quantity of immune system cells from a single donor and they also produced interleukin 2 in amounts exceeding the amounts produced by human peripheral blood lymphocytes [A. Moretta et al., Clin. Exp. Immunol., 44, pp. 262-269 (1981)].

(a) Isolation Of Human Splenocytes

Human spleens obtained from surgical departments were transported to our laboratory in sterile plastic bags and once in the laboratory were transferred to sterile glass dishes. We removed the outer membrane of the spleen with sterile dissection scissors and thus excised 5 cm³ of the spleen tissue. This tissue was placed in a plastic Petri dish (15 cm dia), where it was cut into still smaller pieces (of ~0.5 cm³). After the addition of approximately 10 ml RPMI 1640 medium (Gibco) (supplemented with 2 g/l NaHCO₃ and 20 mM HEPES), we mashed the tissue fragments gently with the end of a plunger from a 2 ml plastic syringe until most of the cells were loosened. We then diluted the suspension with approximately 50 ml RPMI 1640 medium and thoroughly mixed the resulting suspension. We then transferred the tissue and cell suspension to a sterile metal sieve (8 x 0.3 mm/cm) and drained the liquid into another plastic Petri dish. We then returned the tissue on the sieve to the original plastic dish and mashed and filtered as before. Finally, we poured the total cell suspension into a 0.5-1 plastic tube. This procedure was repeated with fresh pieces until the whole spleen had been prepared.

We removed most of the dead cells, which aggregated and formed a thick precipitate on the bottom of the plastic tube, by filtering the suspension again through the metal sieve. We then transferred the cell suspension to 100-ml sterile conical siliconized glass centrifuge tubes and removed any aggregates still present at the bottom of the tubes or at the surface of the suspension.

We then collected the cells by centrifugation at room temperature for 10 min at 900 x g and lysed the erythrocytes by gently suspending the cells in 10 vol cold Tris-NH₄Cl (9 parts 0.83% NH₄Cl plus one part Tris-HCl, (20.6 g/l, pH 7.2)) solution. After 10 min at 4°C, any dead cells that had aggregated at the bottom were removed and the white cells were collected and washed at least twice with RPMI 1640 medium. We then took the cells up in complete medium (RPMI 1640, supplemented with 0.03% L-glutamine, 100 U/ml penicillin, 100 µg/ml streptomycin, 25 µg/ml neomycin, 5 x $10^{-5}$ M β-mercaptoethanol and 10% fetal calf serum) to a concentration of 2-4 x $10^6$ cells/ml.

### (b) Stimulation Of Human Splenocytes With Mitogen

We transferred the above-described cell suspension (~5 l, 1x$10^{10}$ cells) to spinner flasks and added phytohemagglutinin ("PHA") to a concentration of 10 µg/ml (Wellcome) or we added 0.08% PHA-P (Difco) and 10 ng/ml 12-0 Tetradecanoylphorbol 13-acetate ("TPA") (P.L. Biochemicals). After saturation of the atmosphere above the cultures with a 5% $CO_2$ gas mixture, we slowly stirred the suspension at 37°C for 20 h.

We then assayed the suspension for IL2 activity by measuring the uptake of $^3$H-labelled thymidine by IL2 dependent human peripheral blood lymphocytes, as described above.

### (c) Isolation And Translation Of PolyA$^+$ RNA

We collected the PHA/TPA induced splenocytes by centrifugation and washed them with cold PBS. In order to isolate total RNA from these induced splenocytes, we lysed the cells immediately with a solution of guanidinium thiocyanate [J.M. Chirgwin et al., "Isolation Of Biologically Active Ribonucleic Acid From Sources Enriched In Ribonuclease", Biochemistry, 18, pp. 5294-99 (1979)]. We obtained an average of 30 mg total RNA per spleen. We then purified the RNA by precipitation out of a guanidinium hydrochloride solution and by chromatography on oligo(dT) cellulose [J.M. Chirgwin et al., supra]. At this stage about 1 mg (of which more than 60% is rRNA) RNA remained.

We dissolved the resulting polyA$^+$ RNA in sterile water, heated the mixture for 1 min at 68°C and fractionated it on a 5-20% sucrose gradient in 10 mM Tris-HCl (pH 7.5), 1 mM EDTA, employing 1-2 mg RNA per gradient and 40,000 rpm for 16 h at 4°C in a Beckman SW41 Ti rotor. We collected twenty-five fractions (0.4 ml each) in an ISCO gradient fractionator while measuring the optical density (254 nm) continuously.

We assessed the quality of the isolated RNA by measuring the height of the 5S and 18S rRNA peaks (28S rRNA was at the bottom of the tube) after centrifugation in a parallel gradient of approximately 300 µg oligo(dT) cellulose run through RNA.

We precipitated the RNA in each fraction for at least 2 h at -20°C, collected it by centrifugation (10,000 rpm, 30 min, -20°C, HB4 Sorvall rotor) and washed it with 70% ethanol. After the RNA was dried, we dissolved it in 25-50 µl sterile water.

We translated each of the polyA$^+$ RNA sucrose-gradient fractions in wheat germ extract (total vol 10 µl), in the presence of $^{35}$S-methionine [B. Roberts and B.M. Paterson, "Efficient Translation Of TMV RNA And Rabbit Globin 5S RNA In A Cell-Free System From Commercial Wheat Germ", Proc. Natl. Acad. Sci. USA, 70, pp. 2330-34 (1973)] and analyzed the synthesized proteins on a 12.5% SDS-polyacrylamide gel [U.K. Laemmli, "Cleavage Of Structural Proteins During Assembly Of The Head Of Bacteriophage T4", Nature, 227, pp. 680-85 (1970)]. The synthesis of large polypeptides in this system demonstrated that the RNA fractions prepared by us contained RNA that was free of any inhibitors of in vitro translation.

We assayed the polyA$^+$ RNA fractions that sedimented in the sucrose gradient between 7S and 18S to determine the IL2 activity encoded by that mRNA. For this assay, we microinjected 50 µl of polyA$^+$ RNA (1 mg/ml) into each of 15 to 20 Xenopus laevis oocytes and incubated the oocytes for 3 days at 20°C in HEPES buffered, modified Barth solution (MBS-H), containing 0.1% polyethylene glycol and 0.4% aprotinin stock solution (Sigma).

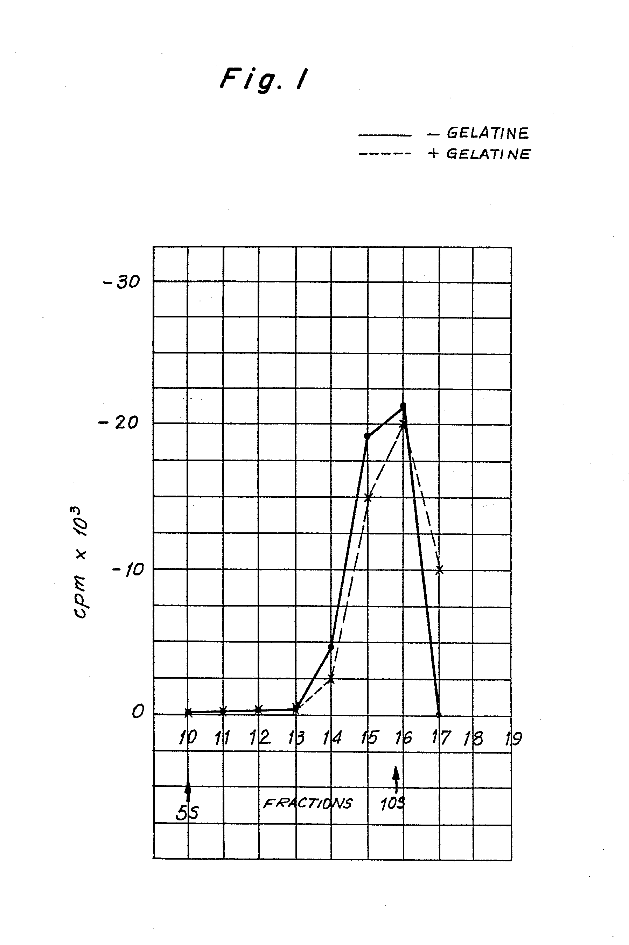

We then withdrew the medium and assayed the secreted translation products for their IL2 activity, as described above. Referring now to Figure 1, we have depicted in a graphical presentation the results of a typical assay experiment. These results demonstrate that the mRNA species encoding human IL2 activity sedimented in the sucrose gradients around 10-11S.

We also examined the kinetics of the secretion of human IL2 by the Xenopus laevis oocytes after microinjection of the sucrose gradient fractionated polyA$^+$ RNA which we isolated from our-mitogen-induced splenocytes. There, we observed that the accumulation of IL2 in the oocyte incubation medium continued for at least 72 h.

At this point it should be recognized that even the poly(A) RNA product obtained from the sucrose gradients contains a very large number of different mRNAs. Except for the mRNA specific for hIL2, the other mRNAs are undesirable contaminants. Unfortunately, these contaminant RNAs behave similarly to hIL2 mRNA throughout the remainder of the cloning process of this invention. Therefore, their presence in the poly(A) RNA will result in the ultimate preparation of a large number of unwanted bacterial clones which contain genes that may code for polypeptides other than IL2. This contamination presents complex screening problems in the isolation of the desired IL2 hybrid clones. In the case of IL2, the screening problem is further exacerbated by the lack of a sufficiently purified sample of IL2 mRNA or DNA or portion thereof to act as a screening probe for the identification of the desired clones. Therefore, the screening process for the IL2 clones is very time-consuming and difficult. Furthermore, because only a very small percentage of IL2 clones themselves are expected to express IL2 in a biologically or immunologically active form, the isolation of an active clone is a "needle in a haystack" screening process.

Advantageously, we may use recombinant DNA technology to provide a purified sample of IL2 mRNA or cDNA or a portion thereof. This purified mRNA or cDNA can then be used to screen rapidly very large numbers of bacterial clones and thereby permits us to isolate a clone which expresses IL2 in an active form.

## SYNTHESIS OF DOUBLE-STRANDED cDNA CONTAINING hIL2 cDNA

Poly(A) RNA enriched in hIL2 mRNA was used as a template to prepare complementary DNA ("cDNA"), essentially as described by R. Devos et al., "Construction And Characterization Of A Plasmid Containing A Nearly Full-Size DNA Copy Of Bacteriophage MS2 RNA", J. Mol. Biol., 128, pp. 595-619 (1979) for the construction of a plasmid containing a DNA copy of bacteriophage MS2 RNA.

We incubated 30 µg polyA$^+$ RNA (three fractions from our sucrose gradients) for 30 min at 43°C in a mixture of 50 µl 50 mM Tris HCl (pH 8.3) 30 mM β-mercaptoethanol, 50 mM KCl, 10 mM MgCl$_2$, 4 dNTPs 0.5 mM each, 10 µg pT$_{12-18}$, 100 µCi α-$^{32}$PdATP and 100 µCi α-$^{32}$PdCTP (2500 Ci/mole each), 4 mM·Na$_4$P$_2$O$_7$ and 100 units AMV reverse transcriptase. We stopped the reaction with EDTA and after phenol extraction loaded the mixture onto a Sephadex-G75 column. We precipitated the nucleic acids in the void fractions with ethanol overnight at -20°C and collected them by centrifugation. We washed the pellet with 70% ethanol, dried it and took it up in 40 μl $H_2O$.

The cDNA population synthesized above is in fact a complex mixture of cDNAs originating from the different mRNAs which were present in the enriched polyA[+] mRNA. In addition, because of premature termination by AMV reverse transcriptase, many of the cDNAs are incomplete copies of the various mRNAs in the polyA[+] RNA.

Before rendering the cDNA double-stranded, it was removed from its association to the complementary template mRNA by heating the above-described water mixture at 100°C for 30 sec and then immediately chilled at 0°C. We added 3 μg Pancreatic RNase and 3 units $T_1$-RNase and incubated the mixture for 30 min at 37°C.

The cDNA strand was rendered double-stranded by adjusting the mixture to 100 μl by addition of a mixture of K-phosphate (pH 6.9) (final concentration 100 mM), dithiothreitol (4 mM), $MgCl_2$ (10 mM), 4dNTPs (250 μM each), 50 μCi $\alpha$-$^{32}$PdATP and 50 μCi $\alpha$-$^{32}$PdCTP (2500 Ci/mole each), adding E. coli DNA polymerase I (Biolabs, 100 units) and incubating the resulting mixture for 3 h at 15°C. The reaction was stopped with EDTA, the mixture phenolized, passed over a Sephadex-G75 column and the void fractions precipitated as before with ethanol at -20°C overnight.

To open the single-stranded hairpin loop which remains on the double-stranded cDNA structure, we removed the DNA by centrifugation, dried the pellet and took it up again in $H_2O$ and incubated the mixture for 30 min at 37°C in 100 μl of 0.2 M NaCl, 50 mM NaOAc (pH 4.5), 1 mM $ZnCl_2$ and 10 units of SI nuclease (Sigma). We stopped the reaction by extracting the mixture with phenol/$CHCl_3$/isoamylalcohol and

precipitated the DNA for 2 h at -20°C and 10 min at -70°C by addition of 200 µl 2 M $NH_4OAc$, 10 µg <u>E. coli</u> tRNA and 1 ml ethanol. We removed the pellet by centrifugation and dried it. This mixture of double-stranded cDNAs is heterogeneous both as a consequence of the heterogeneity of the poly $A^+$ RNA employed as a template to prepare it and because of the probable premature termination of the cDNA transcripts by AMV transcriptase.

To lessen the effect of the latter heterogeneity, we sized the double-stranded cDNA mixture by taking the pellet up in 20 µl 10 mM Tris-HCl (pH 7.5), 1 mM EDTA, incubating it for 5 min at 55°C, adding bromophenol blue-xylenecyanol FF and sucrose and electrophoresing the DNA overnight in a 4% polyacrylamide gel (Tris-borate, 20 cm x 40 cm x 0.3 cm, 200 V-20 mA); a $5'-^{32}P$-labeled <u>Hae</u>III digest of phage $\phi$X174 DNA being electrophoresed in a parallel lane as a marker. After autoradiography of the gel, we separated the DNA into fractions according to their positions on the gel -- M1 (1000-1300 bp), M2 (750-1000 bp), M3 (600-750 bp) and M4 (500-600 bp). We cut out the gel slices corresponding to each fraction and eluted them overnight with 2 ml 0.5 M $NH_4OAc$, 10 mM $MgCl_2$, 0.1% SDS and precipitated the DNA with 2 vol ethanol (at -20°C). After centrifugation, we took each pellet up in 100 µl $H_2O$ and incubated it for 10 min at 37°C in the presence of 900 µl 10 mM Na-phosphate (pH 7.4) and 100 µl (packed vol) hydroxylapatite (Biorad). We then loaded the hydroxylapatite on a Sephadex-G75 column and after washing the column extensively with 2 mM Na-phosphate (pH 7.4), we eluted the DNA with 0.45 M Na-phosphate (pH 7.4) and precipitated it with 1/10 vol 2 M NaOAc (pH 5) and 2 vol ethanol.

Again it must be recognized that within each of our cDNA fractions is a large number of cDNAs, only a very few of which are hIL2-related cDNA.

### CLONING OF DOUBLE-STRANDED cDNA

A wide variety of host/cloning vehicle combinations may be employed in cloning or expressing the double-stranded cDNA prepared in accordance with this invention. For example, useful cloning or expression vehicles may consist of segments of chromosomal, non-chromosomal and synthetic DNA sequences, such as various known derivatives of SV40 and known bacterial plasmids, e.g., plasmids from E. coli including col El, pCR1, pBR322, pMB9 and their derivatives, wider host range plasmids, e.g., RP4, phage DNAs, e.g., the numerous derivatives of phage λ, e.g., NM 989, and other DNA phages, e.g., M13 and Filamenteous single-stranded DNA phages and vectors derived from combinations of plasmids and phage DNAs such as plasmids which have been modified to employ phage DNA or other expression control sequences or yeast plasmids such as the 2 µ plasmid or derivatives thereof. Useful cloning or expression hosts may include bacterial hosts such as E. coli HB 101, E. coli X1776, E. coli X2282, E. coli MRCI and strains of Pseudomonas, Bacillus subtilis, Bacillus stearothermophilus and other bacilli, yeasts and other fungi, animal or plant hosts such as animal (including human) or plant cells in culture or other hosts. Of course, not all host/vector combinations may be equally efficient. The particular selection of host/cloning vehicle combination may be made by those of skill in the art after due consideration of the principles set forth herein without departing from the scope of this invention.

Furthermore, within each specific cloning or expression vehicle, various sites may be selected for insertion of the double-stranded DNA. These sites are usually designated by the restriction endonuclease which cuts them. These sites are well recognized by those of skill in the art. It is, of course, to be understood that a cloning or expression vehicle useful in this invention need not have a restriction endonuclease site for insertion of the chosen DNA fragment. Instead, the vehicle could be joined to the fragment by alternative means.

The vector or cloning or expression vehicle, and in particular the site chosen therein for attachment of a selected DNA fragment to form a recombinant DNA molecule, is determined by a variety of factors, e.g., number of sites susceptible to a particular restriction enzyme, size of the protein to be expressed, susceptibility of the desired protein to proteolytic degradation by host cell enzymes, contamination or binding of the protein to be expressed by host cell proteins difficult to remove during purification, expression characteristics, such as the location of start and stop codons relative to the vector sequences, and other factors recognized by those of skill in the art. The choice of a vector and an insertion site for a particular gene is determined by a balance of these factors, not all selections being equally effective for a given case.

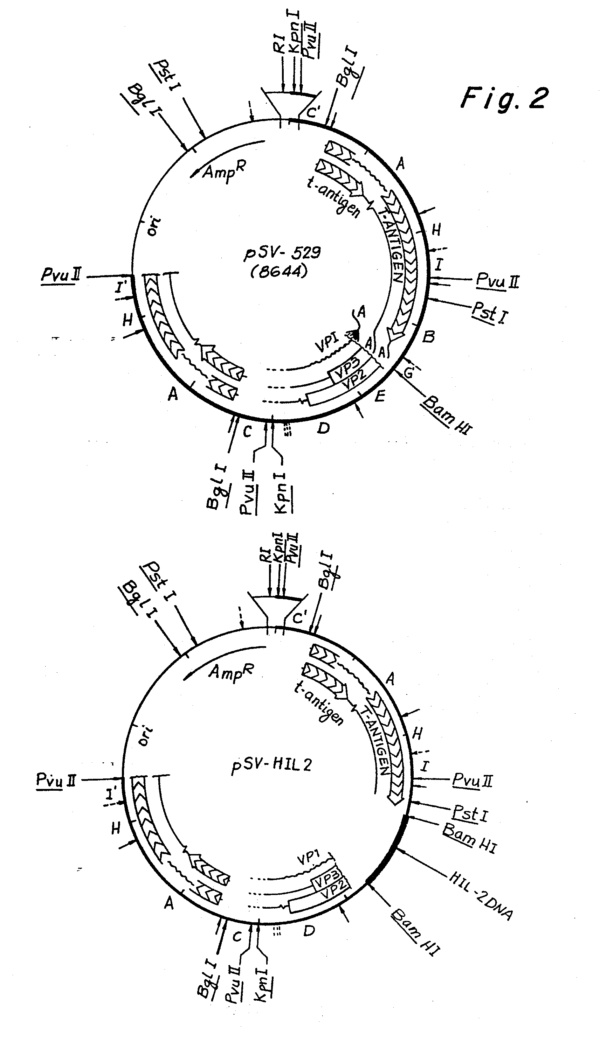

Although several methods are known in the art for inserting foreign DNA into a cloning vehicle or expression vector to form a recombinant DNA molecule, the method preferred for initial cloning in accordance with this invention is digesting pSV529 DNA (infra) with BamHI, filling in the BamHI site and adding dC tails to the 3' termini by terminal transferase. The double-stranded cDNA is then ligated to this pSV529 DNA, after first tailing it with dG tails. The tailed-DNA and tailed-cDNA may then be annealed to insert the DNA in the chosen site of the plasmid and to recircularize the hybrid DNA, the complementary character of the dG-dC tails permitting their cohesion and reconstruction of a BamHI site. The resulting recombinant DNA molecule now carries an inserted gene at the chosen position in the cloning vector (Figure 2).

Of course, other known methods of inserting DNA sequences into cloning or expression vehicles to form recombinant DNA molecules are equally useful in this invention. These include, for example, dA-dT tailing, direct ligation, synthetic linkers, exonuclease and polymerase-linked repair reactions followed by ligation, or extension of the DNA strand with DNA polymerase and an appropriate single-stranded template followed by ligation.

It should, of course, be understood that the nucleotide sequences or cDNA fragments inserted at the selected site of the cloning vehicle may include nucleotides which are not part of the actual gene coding for the desired polypeptide or may include only a fragment of the complete gene for the desired protein. It is only required that whatever DNA sequence is finally inserted, a transformed host will produce a polypeptide having a biological or immunological activity of IL2 or that the DNA sequence itself is of use as a hybridization probe to select clones which contain DNA sequences useful in the production of polypeptides having an immunological or biological activity of IL2.

The cloning vehicle or expression vector containing the foreign gene is employed to transform a host so as to permit that host to express polypeptides displaying an immunological or biological activity of IL2 for which the gene codes. The selection of an appropriate host is also controlled by a number of factors recognized by the art. These include, for example, compatibility with the chosen vector, toxicity of proteins encoded by the hybrid plasmid, ease of recovery of the desired protein, expression characteristics, bio-safety and cost. A balance of these factors must be struck with the understanding that not all hosts may be equally effective for either the cloning or expression of a particular recombinant DNA molecule.

In the present synthesis, the preferred initial cloning vehicle is pSV529 and the preferred initial restriction endonuclease site is BamHI. The preferred initial host is E. coli HB101.

1.    Preparation Of Bam HI-Cleaved, dC-Tailed pSV529 DNA

pSV529 is an chimaeric SV40 plasmid expression vector from which a substantial amount of the late region has been removed (Figure 2). The construction lacks most of the VP1 gene (between 0.945 and 0.145 map units), but retains all the regions which are implicated in replication, initiation and termination of transcription and in splicing and polyadenylation of 16S and 19S mRNA. pSV529 is designed to express a gene under late SV40 transcriptional control [D. Gheysen and W. Fiers, J. Mol. Appl. Genet., 1, pp. 385-94 (1982)].

The main features of pSV529 are: (a) the complete early region of the SV40 genome is present and codes for small-t and large-T antigens as well as the region required for replication in monkey cells, also present are the "enhancer" sequences located towards the late region of ori; (b) the gene for the major structural protein VP1 has been deleted (the HindIII-BamHI fragment from 0.945-0.145 map units); (c) a unique BamHI-site is present on the chimaeric plasmid into which foreign DNA sequences can be inserted for expression under the control of the SV40 late promotor, this BamHI-site is present 39 nucleotides after the late 16S mRNA acceptor splice site and 12 nucleotides before the (now removed) initiation codon of the VP1 gene; (d) donor and acceptor splice sites for the major 16S late message are present; (e) a polyadenylation site from the SV40 late region is present (SV40 map units 0.17); and (f) duplication of a 2080 bp segment of SV40 DNA (between map position 0.33 and 0.725) enables homologous recombination in monkey cells and generates an SV40 replicon from which the plasmid sequences have been deleted but which now contains the inserted gene instead of the viral structural gene VP1.

We digested 50 µg pSV529 DNA with 40 units BamHI restriction enzyme for 2 h at 37°C in 60 µl 8 mM $MgCl_2$, 40 mM NaCl, 100 mM Tris-HCl (pH 7.4). We stopped the reaction with EDTA and the mixture was phenolized (3x), ether extracted (2x) and precipitated with 1/10 vol 2 M KOAc (pH 5) and 2 vol ethanol.

We then separated the precipitated, linearized (Bam HI) pSV529 DNA and washed it (2x) with 70% ethanol, dried it, took it up in $H_2O$ and incubated it for 30 min at 37°C in 50 µl of a mixture of 50 mM Tris-HCl (pH 8.3), 50 mM KCl, 10 mM $MgCl_2$, 30 mM β-mercaptoethanol, 4 dNTPs at 250 µM each and 50 units AMV reverse transcriptase. We stopped the reaction with EDTA and extracted the mixture with phenol/$CHCl_3$/isoamylalcohol. The buffer and mixture were loaded on a Sephadex-G75 column (20 cm x 0.5 cm) in 10 mM Tris-HCl (pH 7.5), 1 mM EDTA and the void fractions precipitated with 2 M KOAc (pH 5) and ethanol at -20°C for 1 h.

We separated the DNA by centrifugation, dried it and took it up in 30 µl 10 mM Tris-HCl (pH 7.5), 1 mM EDTA, heated it for 30 sec at 68°C and chilled it on ice. We then incubated the DNA at 37°C in 200 µl of a mixture of 0.14 M K-cacodylate, 30 mM Tris buffer (pH 6.8), 1 mM $CoSO_4$, 1 mM dithio-

threitol, 0.1 mM dCTP, 100 µCI α-$^{32}$P dCTP (2500 Ci/ mole) and 100 units terminal deoxynucleotidyl transferase (P-L Biochemicals). After 5 min and 10 min, we withdrew aliquots of 100 µl and stopped the reaction with EDTA.

We extracted the aliquots with phenol/CHCl$_3$/ iso-amylalcohol and purified the DNA by chromatography through a Sephadex-G75 column as above. The DNA in the void fractions was precipitated with ethanol, centrifuged, dried and taken up in 30 µl 10 mM Tris-HCl (pH 7.5), 1 mM EDTA. It was calculated that an average of 22 and 36 dCMP residues were added at the 3'-ends of linearized pSV529 DNA after 5 min and 10 min incubation, respectively. Moreover, there was little internal tailing as indicated by agarose gel electrophoresis of the Pvu II digested dC-tailed pSV529 DNA.

### 2. Preparation Of dG-Tailed cDNA Derived From Induced Splenocytes

The double-stranded cDNA (fractions M2 and M3), described above was eluted from the gel, purified on hydroxyapatite and boiled with oligo dG, using conventional methods and terminal dioxynucleotidyl transferase.

### 3. Annealing of dC-elongated pSV529 and dG-elongated cDNA

We mixed the dG-tailed dsDNA from each of fractions M2 and M3 with pSV529 linearized with BamHI filled in and dC-tailed for annealing using standard conditions.

The hybrid DNA obtained after annealing is, of course, a large mixture of different recombinant DNA molecules and some cloning vehicles without inserted DNA sequences. However, each recombinant DNA molecule contains a cDNA segment at the BamHI site. Each such cDNA segment may comprise a gene or a fragment thereof. Only a very few of the cDNA fragments code for IL2 or a portion thereof. The vast majority code for one of the other proteins or portions thereof whose mRNAs were part of the poly(A) RNA used in the process of this invention. It should also be understood that none of the clones of the above-prepared library may permit the expression of polypeptides displaying an immunological or biological activity of IL2. Instead, they may only be useful in screening for and identifying such a clone.

4. Transfection of E. coli HB101
With The Annealed Hybrid DNA

Appropriate containment facilities were employed as necessary for the transfection process and for all subsequent steps in which the resulting transformants were handled.

We added the mixture of annealed DNA (for each of fractions M2 and M3) to competent E. coli HB101 cells using standard transformation conditions. We then plated the cells onto LB-agar plates containing 100 μl/ml carbencillin. Since plasmid pSV529 includes the gene for penicillin resistance, E. coli hosts which have been transformed with a plasmid having that gene intact will grow in cultures containing that antibiotic to the exclusion of those bacteria not so transformed. Therefore, growth in carbencillin-containing culture permits selection of hosts transformed with a recombinant DNA molecule or recyclized vector.

We picked individual colonies and grew them overnight in 200 μl LB-medium (containing carbencillin) in microtiter plates. After adding dimethylsulphoxide to a final concentration of 10%, the plates were stored at -20°C. We obtained approximately 1300 and 1000 clones, respectively, from our M2 and M3 DNA fractions.

The carbencillin-resistant clones of each fraction (M2 and M3) contain a variety of recombinant DNA molecules representing sized, complete or partial copies of the mixture of mRNAs in the poly $A^+$ RNA obtained from the induced splenocytes. The majority of these clones will contain a single recombinant DNA molecule. However, only a very few of these recombinant DNA molecules are related to IL2. Accordingly, the clones must be screened to select the IL2-related clones from the others.

SCREENING FOR A CLONE CONTAINING hIL2 cDNA

There are several approaches to screen for bacterial clones containing hIL2 cDNA. These include, for example, RNA selection hybridization (Alwine et al., infra), differential hybridization (T.P. St. John and R.W. Davis, "Isolation Of Galactose-Inducible DNA Sequences From Saccharomyces Cerevisiae By Differential Plaque Filter Hybridization", Cell, 16, pp. 443-452 (1979)); hybridization with a synthetic probe (B. Noyes et al., "Detection And Partial Sequence Analysis Of Gastrin mRNA By Using An Oligodeoxynucleotide Probe", Proc. Natl. Acad. Sci. USA, 76, pp. 1770-74 (1979)) or screening for clones that produce the desired protein by immunological (L. Villa-Komaroff et al., "A Bacterial Clone Synthesizing Proinsulin", Proc. Natl. Acad. Sci. USA, 75, pp. 3727-31 (1978)) or biological (A.C.Y. Chang et al., "Phenotypic Expression In E. coli Of A DNA Sequence Coding For Mouse Dihydrofolate Reductase", Nature, 275, pp. 617-24 (1978)) assays. We have chosen RNA selection hybridization as being the most convenient and promising method for primary clone screening.

There is no assurance that the recombinant DNA molecules and bacterial cultures transformed therewith, which are identified by RNA selection hybridization, contain the complete IL2 cDNA sequence or even that the DNA sequence actually codes for IL2 or will permit the clone to express polypeptides displaying an immunological or biological activity of IL2. However, the recombinant DNA molecules will certainly contain extensive nucleotide sequences complementary to the IL2 mRNA coding sequence. Therefore, the recombinant DNA molecule may at least be used as a source of a probe to screen rapidly other recombinant DNA molecules and clones transformed with them to identify further sets of clones which will contain an authentic or complete IL2 nucleotide coding sequence. These clones may then be analyzed directly for possible expression of polypeptides displaying a biological or immunological activity of IL2. More importantly, the nucleotide sequence of the inserted DNA fragment of these hybrid plasmids and its amino acid translation product may be determined, using conventional means and that DNA sequence used to construct appropriate expression vectors that permit the synthesis of IL2 in appropriate hosts transformed with them.

A.    RNA Selection Hybridization Assay

    1.    Background of Hybridization Selection

        PolyA$^+$ RNA from our sucrose gradients contains approximately 1 μg RNA/μl of which 30 ng (30 nl) will be microinjected into each oocyte. Assuming that IL2 mRNA constitutes 0.3% of total poly A$^+$ RNA in the sucrose gradient peak fractions, then 0.1 ng IL2 mRNA would be microinjected into each oocyte. One tenth of this amount, i.e., 0.01 ng IL2 mRNA, can be detected after injection into an oocyte. Using the above assumptions, there should be 3 ng IL2 mRNA per μg of polyA$^+$ RNA in the peak fractions and if 50 μg polyA$^+$ RNA is used for the hybridization-elution assay, then there will be 150 ng IL2 mRNA available for hybridization.

Each group of 50 clones contains approximately 10 inserts. 3 to 5 μg of insert DNA is purified by sucrose gradient centrifugation and bound on a nitrocellulose filter. Thus 0.3 to 0.5 μg individual insert DNA (derived from an individual clone) will be bound to the filter.

Assuming 10 ng IL2 mRNA (150 ng is available) can hybridize with 300 to 500 ng IL2 cDNA insert bound to the filter, and assuming 30% of this RNA can be recovered after elution, then 3 ng IL2 mRNA will be taken up in 2 μl of $H_2O$ of which 30 nl or 0.05 ng IL2 mRNA will be injected into an oocyte. This should be detectable in the assay.

Analyzing 30 filters, each containing 10 inserts (derived from 50 clones) will afford us 300 individual inserts to be hybridized. Assuming that IL2 mRNA constitutes 0.3% of the total polyA$^+$ RNA used for ds cDNA synthesis, then on average 1 IL2 cDNA clone should be present in our filters.

2.    Execution of the Assay

Step A:   Purification Of "Insert DNA" And Preparation Of Nitrocellulose Filters Containing That "Insert DNA"

We divided each of the above-prepared libraries (M2 and M3) into groups of 50 clones and grew each set of 50 overnight on a single agar plate. We then suspended the bacteria derived from these clones in 10 ml LB-medium and inoculated 0.5 l Brain Heart Infusion (BHI-Difco) with this suspension. After growing the bacterial suspensions overnight (without amplification of the plasmids), we isolated the DNA from these cultures by SDS-alkali lysis [D. Ish-Horowicz and J.F. Burke, Nucleic Acids Res., 9, pp. 2989-2998 (1981)]. We then digested the plasmid DNA from each mixture of 50 clones (~200 µg) in 200 µl 10 mM Tris-HCl (pH 7.5), 1 mM EDTA with BamHI restriction endonuclease to excise the "insert DNA". After the reaction was complete (checked by electrophoresis of a 5 µl aliquot in a 4% polyacrylamide gel), we heated the DNA mixture at 68°C for 5 min, chilled it in ice and loaded it onto an 11-ml 5-20% sucrose gradient in 10 mM Tris-HCl (pH 7.5), 1 mM EDTA. We centrifuged the gradient for 16 h in a Beckman SW41 rotor at 40K, 4°C.

We then fractionated the gradients and precipitated the fractions containing the mixture of "insert DNA" (~5-10 µg) with 1:10 vol 2 M KOAc (pH 5) and 2 vol ethanol at -20°C for at least 3 h.

We collected the precipitated mixture of "insert DNA" by centrifugation and took up the pellet in a total of 20 µl 10 mM Tris-HCl (pH 7.5), 1 mM EDTA (2 µl was used for electrophoresis on a 4% polyacrylamide gel), heated the mixture for 1 min at 100°C, chilled it in ice, and added 10 µl 1.5 N NaOH. After incubating the mixture for 10 min at room temperature, we added 30 µl 2 M $NH_4OAc$ and spotted it onto a nitrocellulose filter (9 mm$^2$, Schleicher & Schuell BA85, 0.45 µm), premoistened with 1 M $NH_4OAc$, to absorb the "insert DNA" to the filter. We air dried the filters, washed them with 0.9 M NaCl, 0.09 M Na-citrate, dried them and baked them under vacuum overnight at 80°C.

Step B:  Hybridization Of The "Insert-DNA" With PolyA$^+$ RNA

For hybridization selection we prehybridized 30 nitrocellulose filters for 2 h at 50°C in a 15 ml siliconized sterile corex tube containing 500 µl of a mixture of 65% formamide (deionized with Amberlite MB1, Serva), 0.2% SDS, 20 mM HEPES (pH 6.4), 0.4 M NaCl. We then replaced the solution with 500 µl of an identical mixture containing polyA$^+$ RNA (50 µg derived from PHA/TPA-induced splenocytes, purified on a 5-20% sucrose gradient, as described previously and preheated for 1 min at 68°C). We then incubated these filters and mixture for 5 h at 50°C. The 30 nitrocellulose filters represented 30 groups of 50 clones, each prepared from a part of the M2 and M3 fractions, described previously.

<u>Step C:</u>    Separation Of Hybridized PolyA$^+$ RNA-Insert DNA From Non-Hybridized PolyA$^+$ RNA

We transferred the 30 filters containing the hybridized polyA$^+$ RNA-insert DNA to a 50 ml plastic tube and washed them 9 times with 50 ml of a solution of 10 mM Tris-HCl (pH 7.6), 0.15 M NaCl, 1 mM EDTA, 0.5% SDS and twice with the above buffer without SDS at 65°C. We then transferred each individual filter to a siliconized Eppendorf tube containing 100 µl $H_2O$ and 2 µl (4 µg) polyA$^-$ RNA (oligo(dT) cellulose column run through RNA from splenocytes). We heated the tubes for 1 min at 100°C and immediately froze them in a $CO_2$/ethanol bath. After thawing the tubes to room temperature, we removed the filter from each tube and precipitated the eluted RNA overnight at -20°C with 15 µl 2 M NaOAc (pH 5) and 300 µl ethanol. We collected the RNA by centrifugation, washed the pellet twice with 70% ethanol and dried it.

<u>Step D:</u>    Determination Of IL2-mRNA Activity

For assay of the IL2-mRNA activity of the 30 pellets, we took each of the pellets, corresponding to the 30 filters and therefore to the 30 original groups of 50 clones from fractions M2 and M3, up in 2 µl $H_2O$ and injected the RNA solution into each of 15 to 20 <u>Xenopus</u> <u>laevis</u> oocytes, as before. After 3 days at 23°C, the oocyte medium was removed and assayed for IL2 activity (as before).

3.    Results Of The RNA
      <u>Selection Hybridization Assay</u>

Of the 30 groups of 50 clones (18 groups from cDNA fraction M2 (750-1000 bp) and 12 groups from cDNA fraction M3 (600-750 bp)), two groups -- Group M3-2 (Filter 20) and Group M3-6 (Filter 24) -- contained insert DNA that hybridized to mRNA (from polyA[+] RNA from induced splenocytes) that encoded IL2 activity, as measured in the oocyte injection assay.  We have depicted the results of this assay in Table I.

<u>Table I</u>

| <u>Filter No.</u> | <u>Group</u> | <u>cpm*</u> |
|---|---|---|
| 1 | M2-1 | 569 |
| 2 | M2-2 | 533 |
| 3 | M2-3 | 386 |
| 4 | M2-4 | 305 |
| 5 | M2-5 | 293 |
| 6 | M2-6 | 410 |
| 7 | M2-7 | 254 |
| 8 | M2-8 | 333 |
| 9 | M2-9 | 297 |
| 10 | M2-10 | 235 |
| 11 | M2-11 | 253 |
| 12 | M2-12 | 297 |
| 13 | M2-13 | 411 |
| 14 | M2-14 | 263 |
| 15 | M2-15 | 428 |
| 16 | M2-16 | 338 |
| 17 | M2-17 | 302 |
| 18 | M2-18 | 357 |
| 19 | M3-1 | 248 |
| 20 | M3-2 | 7238 |
| 21 | M3-3 | 322 |
| 22 | M3-4 | 329 |
| 23 | M3-5 | 366 |
| 24 | M3-6 | 1719 |
| 25 | M3-7 | 345 |
| 26 | M3-8 | 311 |
| 27 | M3-9 | 397 |
| 28 | M3-10 | 387 |
| 29 | M3-11 | 310 |

*    "cpm" designates $^3$H-thymidine incorporation of 1:4 dilution of oocyte incubation medium by PHA-stimulated IL2 dependent human peripheral blood lymphocytes.

Table I (continued)

| Filter No. | Group | cpm |
|---|---|---|
| 30 | M3-12 | 373 |
| Cl[1] | --- | 11654 |
| C2[2] | --- | 3861 |
| Blank[3] | --- | 314 |
| Standard[4] | --- | 20576 |

After a second hybridization and assay, as described above, groups M3-2 and M3-6 again scored positively. We divided the 50 clones of group M3-2 into 14 subgroups, subgroups A to N, respectively (each group containing 7 clones, except subgroup N which contained 8 clones, in such a way that the presence of a positive clone in the total group should result in two positive subgroups, i.e., using the "square root method"). We then repeated the above-described hybridization and assay procedures using the nitrocellulose filters containing insert-DNA from these 14 subgroups and found that subgroup M3-2D (containing the clones M3-2-4, 11, 18, 25, 32, 39 and 46) and subgroup M3-2L (containing the clones M3-2-29, 30, 31, 32, 33, 34 and 35) displayed IL2 activity. We, therefore, identified the individual clone M3-2-32 as a clone containing a plasmid that had an "insert DNA" complementary to human IL2-mRNA. Accordingly, we isolated, purified and bound the

[1]    "Cl" designates the first control: injection of polyA$^+$ RNA before use in the hybridization-elution assay into Xenopus laevis.

[2]    "C2" designates the second control: injection of polyA$^+$ RNA after hybridization with the nitrocellulose filter (non-hybridized RNA) into Xenopus laevis.

[3]    "Blank" designates 100 μl of the complete medium (RPMI 1640/10% FCS) used in the IL2 assay.

[4]    "Standard" designates a partially purified IL2 preparation from PHA/TPA induced splenocyte cultures.

insert DNA of clone M3-2-32 onto a nitrocellulose filter and repeated the RNA hybridization assay as described above. In this assay individual clone M3-2-32 displayed about 6800 cpm of IL2 activity.

We designated this clone as E. coli HB101 (pSV529(BamHI)/hIL2-0), its recombinant DNA molecule pSV529(BamHI)/hIL2-0 ("pSV-hIL2-0"), and its DNA insert hIL2-0. This nomenclature indicates that the clone and recombinant DNA molecule comprises plasmid pSV529, containing at the BamHI site hIL2-related cDNA, the particular clone being the first identified.

IDENTIFICATION OF CLONES CONTAINING
RECOMBINANT DNA MOLECULES
CROSS-HYBRIDIZING TO hIL2-0

The pSV-hIL2-0 isolated as described above, was used to screen the library of clones previously prepared from cDNA of groups M2 and M3 by colony hybridization [M. Grunstein and D.S. Hogness, "A Method For The Isolation Of Cloned DNA's That Contain A Specific Gene", Proc. Natl. Acad. Sci. USA, 72, pp. 3961-65 (1975)]. This method allows rapid identification of related clones by hybridization of a radioactive probe made from pSV-hIL2-0 to the DNA of lysed bacterial colonies fixed in nitrocellulose filters.

The library of clones stored in microtiter plates, as described above, was replicated on similar size nitrocellulose sheets (0.45 μm pore diameter, Schleicher and Schuell or Millipore), which had been previously boiled to remove detergent, and the sheets placed on LB-agar plates, containing carbencillin (100 μg/μl). Bacterial colonies were grown overnight at 37°C. Lysis and fixation of the bacteria on the nitrocellulose sheets took place by washing consecutively in 0.5 N NaOH (twice for about 7 min), 1 M Tris-HCl (pH 7.5) (about 7 min), 0.5 M Tris-HCl

(pH 7.5) and 1.5 M NaCl (about 7 min), 2 x SSC (0.15 M NaCl, 0.015 M sodium citrate (pH 7.2)) (about 7 min). After thorough rinsing with ethanol and air drying, the sheets were baked at 80°C for 2 h in vacuo and stored at room temperature.

A Hinf restriction fragment specific for the insert DNA hIL2-O fragment served as the probe for colony hybridization. This fragment (~450 base pairs) was purified by electrophoresis of the Hinf digestion products of pSV-hIL2-O in a 4% polyacrylamide gel. After staining the DNA bands with ethidium bromide, the specific fragment was eluted and any residual ethidium bromide was removed by isoamyl alchohol extraction. The specific fragment was then concentrated by precipitation with ethanol and $^{32}$P-labelled by "nick translation" [P.W.J. Rigby et al., "Labeling Deoxyribonucleic Acid To High Specific Activity In Vitro By Nick Translation with DNA Polymerase I", J. Mol. Biol., 113, pp. 237-251 (1977)] by incubation in 50 µl 50 mM Tris-HCl (pH 7.4), 10 mM MgCl$_2$, 20 mM β-mercaptoethanol, containing 2.5 µl each of dCTP, dTTP and dGTP at 400 µM, 100 pmoles α-ATP (Amersham, 2000 Ci/mmole) and 2.5 units of DNA-polymerase I (Boehringer) at 15°C for 45 min. The unreacted deoxynucleoside triphosphates were removed by gel filtration over Sephadex G-75 in TE buffer. The highly $^{32}$P-labelled DNA was precipitated with 0.1 vol of 2 M sodium acetate (pH 5.1) and 2.5 vol of ethanol at 20°C.

Hybridization of the above probe to the filter impregnated DNA was carried out essentially as described by D. Hanahan and M. Meselson, "Plasmid Screening At High Colony Density", Gene, 10, pp. 63-67 (1980). The filters, prepared above, were preincubated for 2 h at 68°C in 0.1% Ficoll, 0.1% polyvinylpyrrolidone, 0.1% bovine serum albumin, 0.15 M NaCl, 0.03 M Tris-HCl (pH 8), 1 mM EDTA, and rinsed with 0.02% Ficoll, 0.02% polyvinylpyrrolidone, 0.02% bovine serum albumin, 0.75 M NaCl, 0.15 M Tris-HCl (pH 8), 5 mM EDTA and 0.5% SDS. The hybridization proceeded overnight at 68°C in a solution identical to the rinsing solution above using the $^{32}$P-labelled probe which had been denatured at 100°C for 5 min prior to use. The hybridized filters were washed twice with 0.3 M NaCl, 0.06 M Tris-HCl (pH 8), 2 mM EDTA for 2 h at 68°C before air drying and autoradiography.

About 1300 clones originating from cDNA fraction M2 and 1050 clones originating from cDNA fraction M3 were screened. As a result of this colony hybridization screening, we identified 1 colony originating from fraction M3 cDNA that strongly hybridized to the IL2 probe. We designated this clone from fraction M3 : pSV-hIL2-1. It came from the M3-6 group that was previously positive in the RNA selection assay.

The DNA insert hIL2-1 had about 230 bp (including its G/C tails). Because this insert hybridized with the internal _Hinf_ fragment of hIL2-0, the insert probably originated by inserting a ds cDNA molecule which was dG-tailed at an internal nick.

It is, of course, evident that this method of clone screening using the hIL2-0 insert DNA or another DNA insert of a clone identified using that insert, as described above, may be employed equally well on other clones containing DNA sequences arising from recombinant DNA technology, synthesis, natural sources or a combination thereof or clones containing DNA sequences related to any of the above DNA sequences by mutation, including single or multiple, base substitutions, insertions, inversions, or deletions. Therefore, such DNA sequences and their identification also fall within this invention. It is also to be understood that DNA sequences, which are not screened by the above DNA sequences, yet which as a result of their arrangement of nucleotides code for the polypeptides coded for by the above DNA sequences also fall within this invention.

In addition, because of the expected homology between the DNA sequence coding for human interleukin 2 and the DNA sequence coding for IL2 from non-human sources, like mouse, swine, chicken, bovine or dog, the DNA sequences of this invention are useful in the selection of the DNA coding for those non-human interleukin 2's and in the cloning and expression of those non-human interleukins for use in immunotherapeutic agents and methods. Finally, the DNA sequences of this invention or oligonucleotides prepared and derived from them may be employed to select other DNA sequences that encode interleukin-related polypeptides that may not be interleukin 2. Those sequences and polypeptides are also part of this invention.

EXPRESSION OF
POLYPEPTIDES DISPLAYING hIL2 ACTIVITY

In order to prepare kidney cells of the African Green Monkey (AP8) for transfection with plasmids containing an hIL-2-related insert-DNA, we maintained the cells in Dulbecco's modified Eagle's minimal medium ("DME") (GIBCO). The medium contained 10% newborn calf serum (GIBCO), 100 units of penicillin per ml and 100 μg of streptomycin per ml. The cell cultures were transformed with plasmid DNA using a modification of the DEAE-dextran method. The following steps were carried out: the confluent cell monolayers were treated exhaustively with trypsin-EDTA for about 1 h and were then dispersed on 17 mm wells (24 wells per plate; Costar) in DME containing 10% newborn calf serum. After 24 h, the cells were washed two times with HEPES buffered minimal essential medium, and the plasmid DNA (dissolved at about 1-10 μg/μl in 12 μl F11-HEPES buffer) was added to 120 μl DME containing 500 μg/ml DEAE-dextran (Pharmacia). This mixture was then applied to the cell monolayers for about 30-60 min [J.H. McCutchan and J.S. Pagano, "Enhancement Of The Infectivity Of Simian Virus 40 Deoxyribonucleic Acid With Diethylaminoethyl-Dextran", J. Natl. Cancer Institute, 41, pp. 351-57 (1978); G. Chu and P.A. Sharp, "SV40 DNA Transfection Of Cells In Suspension: Analysis Of The Efficiency Of Transcription And Translation Of T-Antigen", Gene, 13, pp. 197-202 (1981)]. After the cells had been washed three times with DME, fresh medium (DME + 10% calf serum + 100 µg/ml penicillin and 100 µg/ml streptomycin) was added, and the cell cultures were incubated at 37°C in a $CO_2$ incubator for 72 h.

The plasmid DNA, used for the transfection, was prepared from E. coli HB 101 (pSV-hIL2-0) colonies by a lysozyme-detergent lysis and purified by isodensity centrifugation in CsCl gradients in the presence of ethidium bromide essentially as described by M. Kahn et al., "Plasmid Cloning Vehicles Derived From Plasmids ColEl", in Methods in Enzymology, 68, Recombinant DNA (R. Wu., ed), pp. 268-280 (1979).

After incubation for 72 h, the supernatant (~1/2 ml) was removed from the transformed cells and employed in a conventional IL2 assay to determine the presence of biologically active hIL2-related polypeptides in the supernatant (supra).

We did not observe the expression of any IL2 activity from clone pSV-hIL2-0. Subsequent DNA sequencing (infra) revealed that the DNA insert hIL2-0 of this clone was incomplete at its 5' end and does not include a start signal.* The expression vector

---

* To establish the number of missing nucleotides at the 5' end of hIL2-0, a short (63 bp) restriction fragment was isolated. 5' labelled, strand-separated and extended by reverse transcriptase after hybridization to polyA[+] RNA derived from induced splenocytes. Analysis of the cDNA product on a denaturing polyacrylamide gel showed a band of about 210 nucleotides. From this analysis, we deduced that hIL2-0 was about 110 nucleotides shorter than the full-length IL2 mRNA.

used by us also does not supply a start signal. Therefore, no hIL2 expression could be expected in our expression system using hIL2-O alone. It should, however, be understood that DNA insert hIL2-O may be used to select other clones from the present library of clones or from other libraries or a chromosomal bank which do have the complete coding sequence of IL2 or which will supply the missing parts of that sequence for combination with the sequence of hIL2-O. Such selection techniques are described above and illustrated below. After we have identified a clone having the complete coding sequences for hIL2, that sequence may be employed to transform monkey cells, as described above, or other appropriate hosts to produce hIL2-like polypeptides. In addition, the present hIL2-O sequence may be combined with an ATG start codon by conventional methods to express that sequence or the portions thereof that encode mature hIL2.

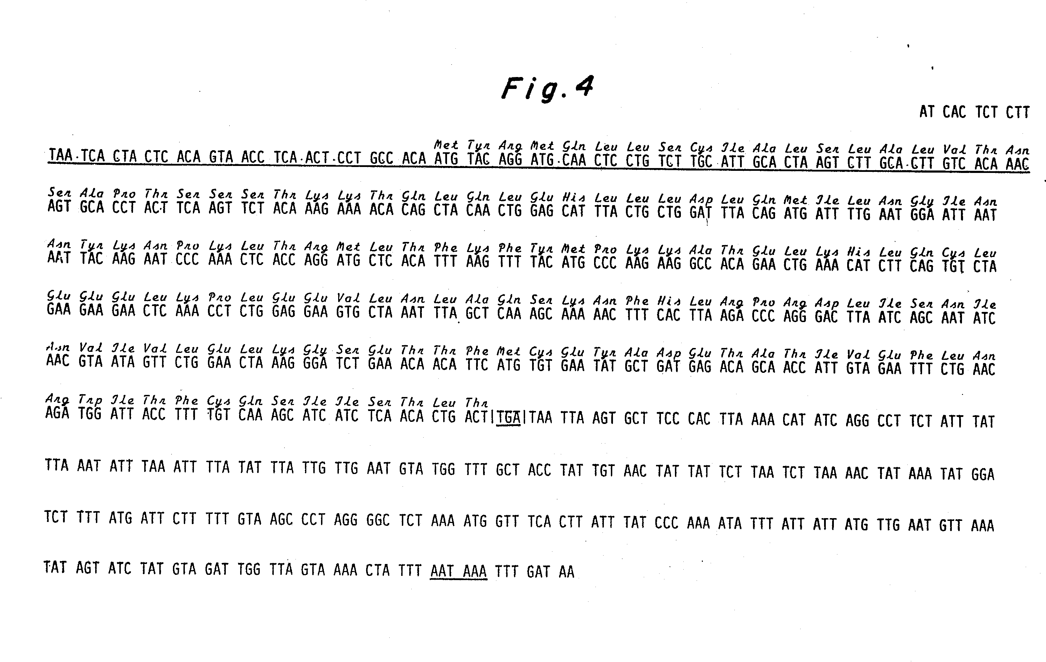

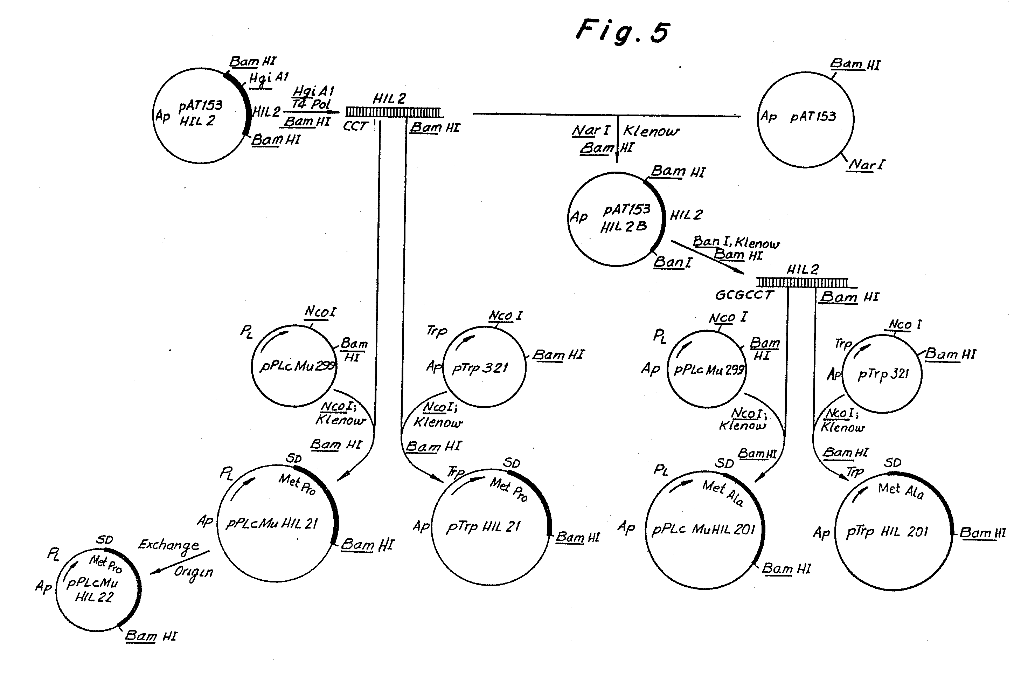

Referring now to Figure 5, we have depicted therein several constructions in which we employed the hIL2-O DNA sequence to produce hIL2. For these construction we initially isolated the BamHI-BamHI fragment of pSV-hIL2-O. This is the fragment (hIL2-O) whose nucleotide sequence we have displayed in Figure 4 (except for the portion underscored).

We first subcloned this fragment into pAT153 (Figure 5) to simplify our manipulations and then linearized the resulting plasmid pAT153 now containing the hIL2-O coding sequence with HgiAl, removed the 3' overhanging ends with T4 polymerase and then restricted the linear DNA with BamHI to isolate an hIL2-containing fragment whose 5' end begins with CCT (the codon for the third amino acid shown in Figure 4) and whose 3' end is beyond the end of the hIL2 coding region (Figure 4). We designated this fragment hIL21.

Because DNA sequence hIL21 codes for a protein product beginning with Pro (the third amino acid of the hIL2-O sequence depicted in Figure 4), we decided to prepare a DNA sequence that also encoded the second amino acid (Ala) of that sequence.\* To effect this construction we first ligated the hIL21 fragment to a fragment that we prepared from pAT153 by linearizing that plasmid with $\underline{\text{Nar}}$I (GG$^{\downarrow}$CGCC), filling in the staggered end with DNA polymerase I (Klenow) (which also forms a $\underline{\text{Ban}}$I site) and restricting the linear fragment with $\underline{\text{Bam}}$HI. The resulting plasmid, designated pAT153 (hIL2B) in Figure 5, has the following sequence at the junction:

GGCGCCT . . .

Pro

We next linearized that plasmid with $\underline{\text{Ban}}$I, filled in the 5' overhanging ends with DNA polymerase I (Klenow) and restricted the linear plasmid with $\underline{\text{Bam}}$HI. We designated the resulting hIL2-containing fragment hIL201. Its 5' end begins with GCGCCT (coding for Ala-Pro) and its 3' end is a $\underline{\text{Bam}}$HI site that is beyond the end of the hIL2 coding sequence. We then inserted these fragments into various expression vectors adjacent to an ATG start codon and downstream of a promoter and ribosome binding site. Such constructions produce an hIL2-related product having as the amino terminal end, Met-Pro-Thr or Met-Ala-Pro-Thr, respectively.

(a)  A $P_L$ Promoter And A Ribosome
     Binding Site Derived From Phage Mu

We inserted the above described sequences into expression vector pPLcMu299 (a gift of Gary Buell). This vector contains the $P_L$ promoter and a

---

\*    Mature hIL2 begins with this Alanine.

ribosome binding site derived from phage Mu.*  It also is characterized by an ATG start codon which is part of an NcoI site (CCATGG) and by a unique BamHI site further downstream.

Accordingly, we linearized pPLcMu299 with NcoI, filled in the '5 staggered ends with DNA polymerase I (Klenow fragment) in the presence of all four dNTP's and further digested the linear DNA with BamHI.  After purification over an agarose gel, we ligated the linearized pPLcMu299 with the above-described hIL2-containing fragments and designated the resulting plasmids pPLcMu-hIL21 and pPLcMu-hIL201 on the basis of which hIL2-containing fragment was inserted.  The DNA sequence at the junction of construction pPLcMu-hIL21 is:

TTAGGAGGGTTTTTACCATGCCT . . .
S.D.          MetPro . . .

The DNA sequence at the junction of construction pPLcMu-hIL201 is:

TTAGGAGGGTTTTTACCATGGCGCCT . . .
S.D.          MetAlaPro . . .

We also prepared a second plasmid characterized by the construction of pPLMu-hIL21, the ribosome binding site from phage Mu and the $P_L$ promoter by exchanging the origin regions of pPLcMuhIL21 with those of pPLc28 [E. Remaut et al., supra].  We designated this smaller plasmid pPLcMu-hIL22 (Figure 5).

We then transformed E. coli K12ΔHlΔtrp with each of these expression vectors and induced

---

*    This vector was derived from pPLc236 [E. Remaut et al., Plasmid Vectors For High-Efficiency Expression Controlled By The $P_L$ Promoter Of Coliphage Lambda", Gene, pp. 81-93 (1981)].  A microorganism and a plasmid containing these sequences were also deposited in the American Type Culture Collection under ATCC accession number 39173 on August 16, 1982. That culture will be made available at the same time the cultures referred to infra are made available.

the resulting cultures at 42°C for 3 h. We then prepared bacterial extracts by disrupting the bacteria in boiling SDS and analyzed the extracts on SDS-polyacrylamide (stained with Serva Blue). In each of the three extracts a new protein band of about 15K was present after induction. However, the band was absent in extracts from transformed cells grown at 28°C. We estimated the 15K protein to be about 5-10% of the total cellular proteins.

We also assayed the hIL2 biological activity of cleared extracts prepared from these trans-formed hosts using the ³H-thymidine incorporation assay described previously. In this assay we opened the cultured and induced cells by sonication in the absence of detergent, spun down the cellular debris at 30000 g and cleared the supernatant through a 0.2 µ Millipore filter. The results of this assay are displayed in Table II.

## Table II

| Sample | | hIL2 (units/ml)* |
|---|---|---|
| Hil2 standard | | 100 |
| Hil2 + 25% control extract | | 70 |
| Hil2 + 2.5% control extract | | 81 |
| pPLcMu-hIL21 | (28°C) | <1 |
| pPLcMu-hIL21 | (42°C) | 25 |
| pPLcMu-hIL22 | (28°C) | <1 |
| pPLcMu-hIL22 | (42°C) | 130 |
| pPLcMu-hIL201 | (28°C) | <1 |
| pPLcMU-hIL201 | (42°C) | 850 |

* These assays are only qualitative with respect to the total amount of protein produced in the trans-formed hosts, because most of the hIL2 remained in the pellet. Therefore, the amount of hIL2 present in the cleared extracts has no relation to the total bacterial synthesis of hIL2.

(b) A Trp Promoter And A Ribosome
Binding Site Derived From The
Trp Attenuation Region

We next inserted the above described hIL2 sequences into expression vector pTrp321 (a gift of Gary Buell). This vector contains the Trp promoter and a ribosome binding site derived from the Trp attenuation regions. The vector part is essentially pBR322, the initiator ATG codon is part of an NcoI site, and there is a unique BamHI site. Accordingly, we followed same construction techniques as described in (a), supra, to prepare plasmids containing the hIL2 sequences. We designated these plasmids pTrp-hIL21 and pTrp-hIL201 on the basis of which hIL2-containing fragment was employed. The DNA sequence at the junction of construction pTrp-hIL21 is:

AAAGGGTATCGATTCCATGCCT . . .
   S.D.            MetPro . . .

The DNA sequence at the junction of construction pTrp-hIL201 is:

AAAGGGTATCGATTCCATGGCGCCT . . .
                 MetAlaPro . . .

We then transformed E. coli K514λ with pTrp-hIL21 and pTrp-hIL201 and induced those transformed hosts at 37°C by trptophane starvation. After induction, we again observed a 15K protein band in the induced cells. As expected, this band was not present in the non-induced cells. Again, the level of inducible synthesis of human interleukin 2 activity was 5 to 10% of total cell proteins.

We again assayed for biological activity after opening the cells of the induced culture by sonication in the absence of detergent, spinning down the cellular debris at 30000 g and passing the supernatant through a 0.2μ millipore filter. The results of these assays are displayed in Table III.

Table III

| Sample | hIL2 (units/one)* |
|---|---|
| Hil2 standard | 100 |
| Hil2 standard + 25% control extract | 70 |
| Hil2 standard + 2.5% control extract | 81 |
| pTrp 321 | <1 |
| pTrp-hIL21 (induction) | 2100 |
| pTrp-hIL201 (induction) | 3800 |

(c)  Recovery Of Biological Activity
     From Bacterial Extracts

We suspended the bacterial pellets, prepared
from 1 ml cultures (pPLcMu-hIL21 or pTrp-hIL21), in
Laemli electrophoresis buffer (200 µl), containing
1% SDS-1% β-mercaptoethanol and incubated the suspension for 1 h at 37°C and then sonicated it.  Following
electrophoresis of these extracts (40 µl) through a
15%-PAGE, containing 0.1% SDS (Laemli), we cut the
gel into 2 mm slices and placed each gel slice into
a microtiter plate well.  We then washed the gel
slices twice with RPMI 1640 medium, containing 10%
fetal calf serum (supplemented with streptomycin
and penicillin), crushed the gels with a glass rod
and eluted them overnight at 37°C by diffusion into
200 µl medium.  After we had incubated the plate at
4°C for 2 h, we centrifuged it at 200 g for 15 min
and assayed the supernatant for hIL2 activity, as
before.

As a result of this assay, we determined
that the hIL2 biological activity of the bacterial
extracts (pPLcMu-hIL21 or pTrp-hIL21) comigrates
with the 15K protein band that appears upon induction
of the transformed cells and that this biological
activity is recoverable from SDS-PAGE.

---

*    Again, these results are only qualitative, because most of the hIL2 activity remains in the non-
assayed pellet, not in the assayed cleared extracts.

## CHARACTERIZATION OF
## hil2- RELATED INSERT DNA

Referring now to Figure 4, a physical map of insert DNA hIL2-0 was constructed by digestion with various restriction enzymes (New England Biolabs or Boehringer) under conditions specified by the suppliers. The products of digestion were electrophoresed in 2.2% agarose or 6% polyacrylamide gels in 40 mM Tris-HOAc (pH 7.8), 20 mM EDTA. They were analyzed after visualization by staining with ethidium bromide or after autoradiography where the restriction fragments were terminally labelled with $^{32}$P-phosphate (infra) and compared with the detailed physical map of pBR322 [J.G. Sutcliffe, "Complete Nucleotide Sequence Of The Escherichia coli Plasmid pBR322", Cold Spring Harbor Symposium, 43, I, pp. 77-90 (1978)]. Restriction maps were constructed on the basis of these digestion patterns. These were refined by sequencing the DNA inserts, substantially by the procedure of A.M. Maxam and W. Gilbert, "A New Method For Sequencing DNA", Proc. Natl. Acad. Sci. USA, 74, pp. 560-64 (1977). The sequence of hIL2-0 was supplemented by sequence information from a genomic hIL2 clone described infra. The genomic-derived information is underscored in Figure 4.

Plasmid DNA was prepared from pSV-hIL2-0 by the method of Ish-Horowicz and Burke (supra), employed previously herein to isolate the DNA from the sets of clones for screening. The insert DNA was obtained by BamHI restriction of pSV-hIL2-0 and was purified by neutral sucrose gradient centrifugation as before (about 740 bp). It was then restricted by various restriction enzymes, essentially as recommended by the supplier (New England Biolabs).

Restricted DNA was dephosphorylated for 30 min at 65°C in the presence of 4 units bacterial alkaline phosphatase and 0.1% SDS. Following two phenol extractions and ethanol precipitation, the DNA was 5'-terminally labelled with $\gamma$-$^{32}$P-ATP (~3000 Ci/mmole) and polynucleotide kinase (P-L Biochemicals, Inc.). Alternatively, restriction fragments were 3'-terminally labelled by limited DNA-polymerase reaction (Klenow fragment, Boehringer) in the presence of an $\alpha$-labelled $^{32}$P-nucleoside triphosphate (~ 1000 Ci/mmole).

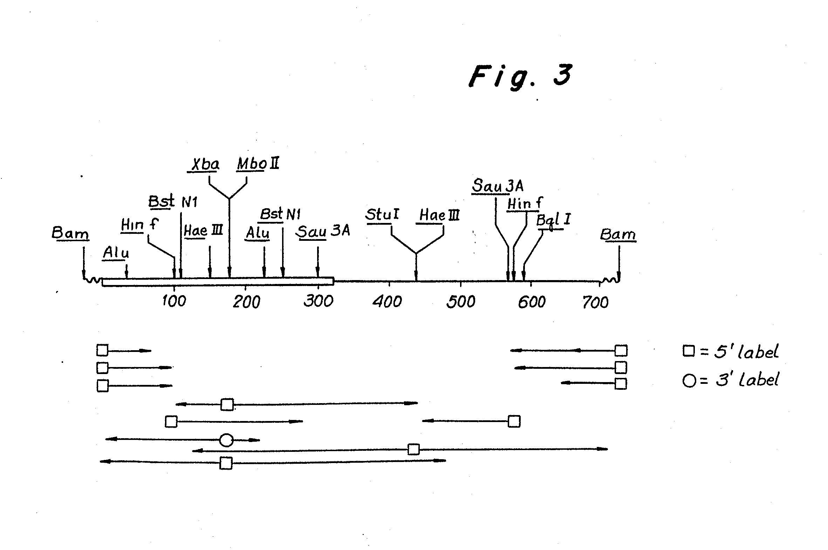

For sequencing, labelled fragments were handled in two ways. Some were purified on a polyacrylamide gel prior to cleavage with a second restriction enzyme. Others were immediately cleaved with a second restriction enzyme. In both cases the desired fragments were separated on a polyacrylamide gel in Tris-borate-EDTA buffer. Figure 3 displays the various restriction fragments (the circles and squares indicating the 3' and 5' labels, respectively, and the arrows the direction of sequencing) and the sequencing strategy which we employed. No restriction sites were found for the enzymes AccI, Apa, AvaI, BglII, BstEI, DdeI, EcoRI, EcoRV, Fnn4HI, HaeII, HincII, HpaI, HphI, KpnI, MspI, NaeI, NruI, PvuI, PvuII, SacI, SmaI, SphI and Taq.

The fragments were degraded according to the method of A.M. Maxam and W. Gilbert (supra). The products were fractionated on polyacrylamide gels of various concentrations and lengths in 50 mM Tris-borate, 1 mM EDTA (pH 8.3) at 900 V to 2000 V. The composite nucleotide sequence thus obtained and its corresponding amino acid sequence is depicted in Figure 4.

The DNA sequence depicted in Figure 4 for hIL2-0 has a polyadenylation signal AATAAA at positions -9 to -14 from the GC-tails. It has an open reading frame encoding 133 amino acids ending with a termination signal. On the basis of hIL2's molecular weight and assuming that native hIL2 is glycosylated, we had expected that mature hIL2 would have between 100 and 130 amino acids. Therefore, we believe that insert hIL2-0 includes the complete coding sequence for mature hIL2, but that it is missing at least part of the coding region for the amino acids of the putative signal sequence of pre hIL2 and the ATG start signal of that signal sequence.* Again, clones coding for these missing amino acids may be selected or constructed as described previously. For example, in Figure 4, this portion was supplied by sequence information from a genomic clone (underscored). However, the sequence still permits the synthesis of hIL2-like polypeptides in hosts transformed with it.

This sequence does not exclude the possibility that modifications to the gene such as mutations, including single or multiple, base substitutions, deletions, insertions, or inversions may not have already occurred in the gene or may not be employed subsequently to modify its properties or the properties of the polypeptides expressed therefrom. Nor does it exclude any polymorphism which may result in physiologically similar but structurally slightly different genes or polypeptides than that reported in Figures 4.

It should, of course, be understood that cloned cDNA from poly A RNA by the usual procedures [supra] may lack 5'-terminal nucleotides and may even contain artifactual sequences [R.I. Richards et al., "Molecular Cloning And Sequence Analysis Of Adult Chicken β-Globin cDNA", Nucleic Acids Research, 7, pp. 1137-46 (1979)].

---

* We believe that the putative signal peptide cleaved from mature hIL2 between Ser and Ala as indicated by the arrow in Figure 4.

In addition, in eukaryotic mRNAs the first AUG triplet from the 5' terminus is usually the initiation site for protein synthesis [M. Kozak, "How Do Eukaryotic Ribosomes Select Initiation Regions In Messenger RNA?", Cell, 15, pp. 1109-25 (1978)].

The structure of the polypeptide depicted in Figure 4, of course, does not take into account any modifications to the polypeptide caused by its interaction with in vivo enzymes, e.g., glycosylation. Therefore, it must be understood that the amino acid sequence depicted in Figure 4 may not be identical with IL2 produced in vivo.

## IDENTIFICATION OF A
## CHROMOSOMAL GENE CODING FOR IL2

A collection of hybrid phage derived from fragments of fetal human chromosomal DNA which had been generated by partial cleavage with HaeIII and AluI, and joined with EcoRI linkers to λ Charon 4A arms has been prepared by R.M. Lawn et al., Cell, 15, pp. 1157-74 (1978). Analogously, human chromosomal DNA fragments generated by partial cleavage with MboI were cloned in the BamHI sites of λ47 [W.A.M. Loenen and W.J. Brammer, Gene, 20, pp. 249-59 (1980); J. Delamarter, personal communication]. Both gene banks screened by an "in situ" procedure [W.D. Benton and R.W. Davis, Science, 196, pp. 180-82 (1977); T. Maniatis et al., Cell, 15, pp. 687-701 (1978)]; using as a probe the $^{32}$P-labelled HinfI fragment of pSV-hIL2-0, described previously.

We isolated two hybridization-positive phage clones from 1,300,000 plaques of the Lawn gene bank and three from 700,000 plaques of Loenen gene bank. We repeatedly purified these plaques and designated them λCH4A-ghIL2-1, λCH4A-ghIL2-2, and λL47-ghIL2-1, λL47-ghIL2-2 and λL47-ghIL2-3, respectively.

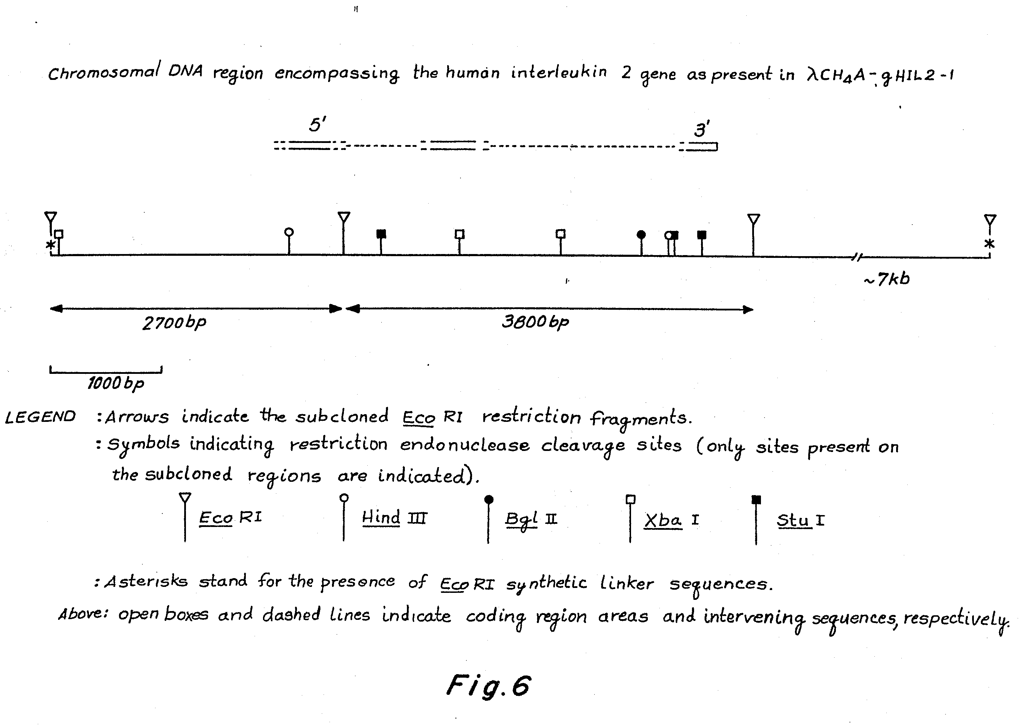

Referring now to Figure 6, we have depicted therein a partial restriction map of plaque λCH4A-ghIL2-1 which contained the total hIL2 gene. We also subcloned this gene in pUR250 [U. Ruther, "pUR Allows Rapid Chemical Sequencing Of Both DNA Strands Of Its Inserts", Nucleic Acids Res., 10, pp. 5765-72 (1981)] as two EcoRI fragments of 2.7kb and 3.8kb, respectively. On the basis of restriction analysis and hybridization data, we determined that pUR-ghIL2-1/2700bp RI-RI contained the signal sequence and the promoter region of hIL2. Our restriction analyses of that plasmid and pUR-ghIL2-1/ 3800bp RI-RI also suggested that there were four exons and three introns present in the hIL2 coding region.

It should be understood that while the chromosomal gene encoding IL2 activity may not be expressible in bacterial hosts because these intervening sequences may not be processed correctly by such hosts, the chromosomal genes are likely to be very useful in the production of IL2 in eukaryotic hosts where the human noncoding regions, introns and coding regions may be important for high levels of expression and correct processing of the product to biologically active IL2.

IMPROVING THE YIELD AND ACTIVITY OF
POLYPEPTIDES DISPLAYING IL2 ACTIVITY
PRODUCED IN ACCORDANCE WITH THIS INVENTION

The level of production of a protein is governed by three major factors: the number of copies of its gene within the cell, the efficiency with which those gene copies are transcribed and the efficiency with which they are translated. Efficiency of transcription and translation (which together comprise expression) is in turn dependent upon nucleotide sequences, normally situated ahead of the desired coding sequence. These nucleotide sequences or expression control sequences define, inter alia, the location at which RNA polymerase interacts to initiate transcription (the promoter sequence) and at which ribosomes bind and interact with the mRNA (the product of transcription) to initiate translation. Not all such expression control sequences function with equal efficiency. It is thus of advantage to separate the specific coding sequences for the desired protein from their adjacent nucleotide sequences and to fuse them instead to other known expression control sequences so as to favor higher levels of expression. This having been achieved, the newly engineered DNA fragments may be inserted into higher copy number plasmids or bacteriophage derivatives in order to increase the number of gene copies within the cell and thereby further to improve the yield of expressed protein.

Several expression control sequences may be employed as described above. These include the operator, promoter and ribosome binding and interaction sequences (including sequences such as the Shine-Dalgarno sequences) of the lactose operon of E. coli ("the lac system"), the corresponding sequences of the tryptophan synthetase system of E. coli ("the trp system"), the major operator and promoter regions of phage λ ($O_L P_L$ as described above and $O_R P_R$), a control region of Filamentous single-stranded DNA phages, or other sequences which control the expression of genes of prokaryotic or eukaryotic cells and their viruses or combinations thereof. Therefore, to improve the production of a particular polypeptide in an appropriate host, the gene coding for that polypeptide may be prepared as before and inserted into a recombinant DNA molecule closer to its former expression control sequence or under the control of one of the above improved expression control sequences. Such methods are known in the art.

Other methods to improve the efficiency of translation involve insertion of chemically or enzymatically prepared oligonucleotides in front of the initiating codon. By this procedure a more optimal primary and secondary structure of the messenger RNA can be obtained. More specifically, a sequence can be so designed that the initiating AUG codon occurs in a readily accessible position (i.e., not masked by secondary structure) either at the top of a hairpin or in other single-stranded regions. Also the position and sequence of the aforementioned Shine-Dalgarno segment can likewise be optimized. The importance of the general structure (folding) of the messenger RNA has been documented [D. Iserentant and W. Fiers "Secondary Structure Of mRNA And Efficiency Of Translation Initiation", Gene, 9, 1-12 (1980].

Further increases in the cellular yield of the desired products depend upon an increase in the number of genes that can be utilized in the cell. This may be achieved by insertion of the IL2 gene (with or without its transcription and translation control elements) in a higher copy number plasmid or in a temperature-controlled copy number plasmid (i.e., a plasmid which carries a mutation such that the copy number of the plasmid increases after shifting up the temperature [B. Uhlin et al. "Plasmids With Temperature-Dependent Copy Number For Amplification Of Cloned Genes And Their Products", Gene, 6, 91-106 (1979)]).

Alternatively, an increase in gene dosage can be achieved for example by insertion of recombinant DNA molecules engineered in the way described previously into the temperate bacteriophage λ, most simply by digestion of the plasmid with a restriction enzyme, to give a linear molecule which is then mixed with a restricted phage λ cloning vehicle

[e.g., of the type described by N. E. Murray et al., "Lambdoid Phages That Simplify The Recovery Of In Vitro Recombinants", Mol. Gen. Genet., 150, 53-61 (1977) and N. E. Murray et al., "Molecular Cloning Of The DNA Ligase Gene From Bacteriophage T4", J. Mol. Biol., 132, 493-505 (1979)] and the recombinant DNA molecule produced by incubation with DNA ligase. The desired recombinant phage is then selected as before and used to lysogenize a host strain of E. coli.

Therefore, it should be understood that the insert DNA of this invention may be removed from the SV529 plasmid and inserted into other expression vectors, as previously described (supra) and these vectors employed in various hosts, as previously described (supra) to improve the expression of the gene coding for IL2.

The biological activity of the IL2-like polypeptides produced in accordance with this invention may also be improved by using the DNA sequences of this invention to transform mammalian cell systems and to express the gene in those systems. Such mammalian systems are known [e.g., P.J. Southern & P. Berg, J. Mol. Appl. Genet., 1, pp. 327-41 (1982); S. Subramani et al., Mol. Cell. Biol., 1, pp. 854-64 (1981); R.J. Kaufman & P.A. Sharp, Mol. Cell. Biol., (in press)]. The preferred system is the CHO (Chinese Hamster ovary) (DHFR⁻) cell system in which the gene expression may be amplified by methotrexate (MTX). These expression systems permit the production of glycosylated proteins.

It should also be understood that polypeptides displaying IL2 activity (prepared in accordance with this invention) may also be prepared in the form of a fused protein (e.g., linked to a prokaryotic or eukaryotic N-terminal segment directing excretion), in the form of prointerleukin 2 (e.g.,

starting with all or parts of the interleukin 2 signal sequence which could be cleaved off upon excretion) or as mature interleukin 2 (by cleavage of any extraneous amino acids, including an initial methionine during expression and excretion) or in the form of a f-met-IL2. One particularly useful polypeptide in accordance with this invention would be mature interleukin 2 with an easily cleaved amino acid or series of amino acids attached to the amino terminus. Such construction would allow synthesis of the protein in an appropriate host, where a start signal not present in mature interleukin 2 is needed, and then cleavage of the extra amino acids to produce mature interleukin 2.

The yield of these different forms of polypeptide may be improved by any or a combination of the procedures discussed above. Also different codons for some or all of the codons used in the present DNA sequences could be substituted. These substituted codons may code for amino acids identical to those coded for by the codons replaced but result in higher yield of the polypeptide. Alternatively, the replacement of one or a combination of codons leading to amino acid replacement or to a longer or shorter IL2-related polypeptide may alter its properties in a useful way (e.g., increase the stability, increase the solubility, increase the immunotherapeutic activity, increase the activity for long-term proliferation of T-cells).

Finally, the activity of the polypeptides produced by the recombinant DNA molecules of this invention may be improved by fragmenting, modifying or derivatizing the DNA sequences or polypeptides of this invention by well-known means, without departing from the scope of this invention. For example, hybrids with other of the interferons might be prepared on the gene level and expressed in appropriate hosts or on the protein level by chemical synthetic methods.

Microorganisms and recombinant .DNA molecules prepared by the processes described herein are exemplified by cultures deposited in the culture collection of the Deutsche Sammulung von Mikroorganism in Gottingen, West Germany, on February 8, 1983:

hIL2-A:  E. coli HB101 (pSV-hIL2-0)

hIL2-B:  E. coli HB101 (pSV-hIL2-1)

These cultures have been assigned accession numbers DSM 2595 and 2596, respectively.

We have also deposited microorganisms and recombinant DNA molecules prepared by the processes described herein in the culture collection of the American Type Culture Collection, Rockville, Maryland on April 18, 1983:

hIL2-C:  E. coli K514λ (pTrp-hIL201)

hIL2-D:  E. coli K12RRIΔM15(λCH4A-ghIL2-1/ 2700bp RI-RI)

hIL2-E:  E. coli K12RRIΔM15((λCH4A-ghIL2-1/ 3800bp RI-RI)

These cultures have been assigned accession numbers ATCC 39338, 39339 and 39340, respectively.

While we have hereinbefore presented a number of embodiments of this invention, it is apparent that .our basic construction can be altered to provide other embodiments which utilize the processes and compositions of this invention.  Therefore, it will be appreciated that the scope of this invention is to be defined by the claims appended hereto rather than by the specific embodiments which have been presented hereinbefore by way of example.

CLAIMS

1. A DNA sequence selected from the group consisting of the DNA insert of pSV-hIL2-0, DNA sequences which hybridize to the foregoing DNA insert and which encode IL2-like polypeptides, and DNA sequences which on expression code for a polypeptide coded on expression for by any of the foregoing DNA sequences and inserts.

2. The DNA sequence according to claim 1, wherein the DNA sequence which hybridizes to said DNA insert is selected from the group consisting of the DNA inserts of pSV-hIL2-1, DNA sequences which hybridize to any of the foregoing DNA inserts and which encode IL2-like polypeptides, and DNA sequences which code on expression for a polypeptide coded for on expression by any of the foregoing DNA sequences and inserts.

3. The DNA sequence according to claim 1, wherein the DNA sequence which hybridizes to said DNA insert is a DNA sequence selected from a human chromosomal gene bank.

4. The DNA sequence according to claim 3, wherein said sequence is selected from the group consisting of the hIL2-related portions of λCH4A-ghIL2-1, λCH4A-ghIL2-2, λL47-ghIL2-1, λL47-ghIL2-2 and λL47-ghIL2-3.

5. The DNA sequence according to any one of claims 1 to 4 selected from the group consisting of DNA sequences of the formula:

AGTGCACCTACTTCAAGTTCTACAAAGAAAACACAGCTACAACTGGAGCATTTA
CTGCTGGATTTACAGATGATTTTGAATGGAATTAATAATTACAAGAATCCCAAA
CTCACCAGGATGCTCACATTTAAGTTTTACATGCCCAAGAAGGCCACAGAACTG
AAACATCTTCAGTGTCTAGAAGAAGAACTCAAACCTCTGGAGGAAGTGCTAAAT
TTAGCTCAAAGCAAAAACTTTCACTTAAGACCCAGGGACTTAATCAGCAATATC
AACGTAATAGTTCTGGAACTAAAGGGATCTGAAACAACATTCATGTGTGAATAT
GCTGATGAGACAGCAACCATTGTAGAATTTCTGAACAGATGGATTACCTTTTGT
CAAAGCATCATCTCAACACTGACT, GCACCTACTTCAAGTTCTACAAAGAAA

ACACAGCTACAACTGGAGCATTTACTGCTGGATTTACAGATGATTTTGAATGG
AATTAATAATTACAAGAATCCCAAACTCACCAGGATGCTCACATTTAAGTTTT
ACATGCCCAAGAAGGCCACAGAACTGAAACATCTTCAGTGTCTAGAAGAAGAA
CTCAAACCTCTGGAGGAAGTGCTAAATTTAGCTCAAAGCAAAAACTTTCACTT
AAGACCCAGGGACTTAATCAGCAATATCAACGTAATAGTTCTGGAACTAAAGG
GATCTGAAACAACATTCATGTGTGAATATGCTGATGAGACAGCAACCATTGTA
GAATTTCTGAACAGATGGATTACCTTTTGTCAAAGCATCATCTCAACACTGACT,
ATGGCACCTACTTCAAGTTCTACAAAGAAAACACAGCTACAACTGGAGCATTT
ACTGCTGGATTTACAGATGATTTTGAATGGAATTAATAATTACAAGAATCCCA
AACTCACCAGGATGCTCACATTTAAGTTTTACATGCCCAAGAAGGCCACAGAA
CTGAAACATCTTCAGTGTCTAGAAGAAGAACTCAAACCTCTGGAGGAAGTGCT
AAATTTAGCTCAAAGCAAAAACTTTCACTTAAGACCCAGGGACTTAATCAGCA
ATATCAACGTAATAGTTCTGGAACTAAAGGGATCTGAAACAACATTCATGTGT
GAATATGCTGATGAGACAGCAACCATTGTAGAATTTCTGAACAGATGGATTAC
CTTTTGTCAAAGCATCATCTCAACACTGACT, ATGAGTGCACCTACTTCAAG
TTCTACAAAGAAAACACAGCTACAACTGGAGCATTTACTGCTGGATTTACAGA
TGATTTTGAATGGAATTAATAATTACAAGAATCCCAAACTCACCAGGATGCTC
ACATTTAAGTTTTACATGCCCAAGAAGGCCACAGAACTGAAACATCTTCAGTG
TCTAGAAGAAGAACTCAAACCTCTGGAGGAAGTGCTAAATTTAGCTCAAAGCA
AAAACTTTCACTTAAGACCCAGGGACTTAATCAGCAATATCAACGTAATAGTT
CTGGAACTAAAGGGATCTGAAACAACATTCATGTGTGAATATGCTGATGAGAC
AGCAACCATTGTAGAATTTCTGAACAGATGGATTACCTTTTGTCAAAGCATCA
TCTCAACACTGACT, and DNA sequences which on expression
code for a polypeptide coded for on expression by any
of the foregoing DNA sequences.

6.   The DNA sequence according to any one
of claims 1 to 4 selected from the group consisting
of DNA sequences of the formula:
CCTACTTCAAGTTCTACAAAGAAAACACAGCTACAACTGGAGCATTTACTGCT
GGATTTACAGATGATTTTGAATGGAATTAATAATTACAAGAATCCCAAACTCA
CCAGGATGCTCACATTTAAGTTTTACATGCCCAAGAAGGCCACAGAACTGAAA
CATCTTCAGTGTCTAGAAGAAGAACTCAAACCTCTGGAGGAAGTGCTAAATTT
AGCTCAAAGCAAAAACTTTCACTTAAGACCCAGGGACTTAATCAGCAATATCA
ACGTAATAGTTCTGGAACTAAAGGGATCTGAAACAACATTCATGTGTGAATAT
GCTGATGAGACAGCAACCATTGTAGAATTTCTGAACAGATGGATTACCTTTTG
TCAAAGCATCATCTCAACACTGACT, ATGCCTACTTCAAGTTCTACAAAGAA
AACACAGCTACAACTGGAGCATTTACTGCTGGATTTACAGATGATTTTGAATG

GAATTAATAATTACAAGAATCCCAAACTCACCAGGATGCTCACATTTAAGTTT
TACATGCCCAAGAAGGCCACAGAACTGAAACATCTTCAGTGTCTAGAAGAAGA
ACTCAAACCTCTGGAGGAAGTGCTAAATTTAGCTCAAAGCAAAAACTTTCACT
TAAGACCCAGGGACTTAATCAGCAATATCAACGTAATAGTTCTGGAACTAAAG
GGATCTGAAACAACATTCATGTGTGAATATGCTGATGAGACAGCAACCATTGT
AGAATTTCTGAACAGATGGATTACCTTTTGTCAAAGCATCATCTCAACACTGA
CT and DNA sequences which on expression code for a polypeptide coded for on expression by any of the foregoing DNA sequences.

7. The recombinant DNA molecule comprising a DNA sequence being selected from the group consisting of DNA sequences according to any one of claims 1 to 6.

8. The recombinant DNA molecule according to claim 7, wherein said DNA sequence is operatively linked to an expression control sequence.

9. The recombinant DNA molecule according to claim 8, wherein said expression control sequence is selected from the group consisting of a lac system, a β-lac system, a trp system, major operator and promotor regions of phage λ, the control region of fd coat protein, other sequences which control the expression of genes of prokaryotic or eukaryotic cells and their viruses and combinations thereof.

10. The recombinant DNA molecule according to claim 7 selected from the group consisting of pPLcMu-hIL21, pPLcMu-hIL22, pPLcMu-hIL201, pTrp-hIL21, and pTrp-hIL201.

11. A host transformed with at least one recombinant DNA molecule according to any one of claims 7 to 10.

12. The host of claim 11 selected from the group consisting of strains of E. coli, Pseudomonas, Bacillus subtilis, Bacillus stearothermophilus, other bacilli, yeast, other fungi, mouse or other animal or plant hosts and human tissue cells.

13. The transformed host according to claim 11 or 12 selected from the group consisting of strains of <u>E. coli</u>, <u>Pseudomonas</u>, <u>Bacillus</u> <u>subtilis</u>, <u>Bacillus</u> <u>stearothermophilus</u>, other bacilli, yeasts, other fungi, mouse or other animal or plant hosts or human tissue cells transformed with a DNA sequence selected from the group consisting of the sequences of claims 1 to 6.

14. A polypeptide displaying an immunological or biological activity of human interleukin 2 produced by the transformed host according to any one of claims 11 to 13.

15. A polypeptide that is coded for by a DNA sequence according to any one of claims 1 to 6.

16. The polypeptide of claim 15 coded for by the DNA insert hIL2-0.

17. A polypeptide according to claims 14 to 16 selected from the group consisting of polypeptides of the formula:

AlaProThrSerSerSerThrLysLysThrGlnLeuGlnLeuGluHisLeuLeu
LeuAspLeuGlnMetIleLeuAsnGlyIleAsnAsnTyrLysAsnProLysLeu
ThrArgMetLeuThrPheLysPheTyrMetProLysLysAlaThrGluLeuLys
HisLeuGlnCysLeuGluGluGluLeuLysProLeuGluGluValLeuAsnLeu
AlaGlnSerLysAsnPheHisLeuArgProArgAspLeuIleSerAsnIleAsn
ValIleValLeuGluLeuLysGlySerGluThrThrPheMetCysGluTyrAla
AspGluThrAlaThrIleValGluPheLeuAsnArgTrpIleThrPheCysGln
SerIleIleSerThrLeuThr, ProThrSerSerSerThrLysLysThrGln
LeuGlnLeuGluHisLeuLeuLeuAspLeuGlnMetIleLeuAsnGlyIleAsn
AsnTyrLysAsnProLysLeuThrArgMetLeuThrPheLysPheTyrMetPro
LysLysAlaThrGluLeuLysHisLeuGlnCysLeuGluGluGluLeuLysPro
LeuGluGluValLeuAsnLeuAlaGlnSerLysAsnPheHisLeuArgProArg
AspLeuIleSerAsnIleAsnValIleValLeuGluLeuLysGlySerGluThr
ThrPheMetCysGluTyrAlaAspGluThrAlaThrIleValGluPheLeuAsn
ArgTrpIleThrPheCysGlnSerIleIleSerThrLeuThr, MetAlaPro
ThrSerSerSerThrLysLysThrGlnLeuGlnLeuGluHisLeuLeuLeuAsp
LeuGlnMetIleLeuAsnGlyIleAsnAsnTyrLysAsnProLysLeuThrArg
MetLeuThrPheLysPheTyrMetProLysLysAlaThrGluLeuLysHisLeu

GlnCysLeuGluGluGluLeuLysProLeuGluGluValLeuAsnLeuAlaGln
SerLysAsnPheHisLeuArgProArgAspLeuIleSerAsnIleAsnValIle
ValLeuGluLeuLysGlySerGluThrThrPheMetCysGluTyrAlaAspGlu
ThrAlaThrIleValGluPheLeuAsnArgTrpIleThrPheCysGlnSerIle
IleSerThrLeuThr, MetProThrSerSerSerThrLysLysThrGlnLeu
GlnLeuGluHisLeuLeuLeuAspLeuGlnMetIleLeuAsnGlyIleAsnAsn
TyrLysAsnProLysLeuThrArgMetLeuThrPheLysPheTyrMetProLys
LysAlaThrGluLeuLysHisLeuGlnCysLeuGluGluGluLeuLysProLeu
GluGluValLeuAsnLeuAlaGlnSerLysAsnPheHisLeuArgProArgAsp
LeuIleSerAsnIleAsnValIleValLeuGluLeuLysGlySerGluThrThr
PheMetCysGluTyrAlaAspGluThrAlaThrIleValGluPheLeuAsnArg
TrpIleThrPheCysGlnSerIleIleSerThrLeuThr and hIL2-like
fragments thereof.

18.    A method for producing a recombinant
DNA molecule comprising the step of introducing into
a cloning vehicle a DNA sequence according to any
one of claims 1 to 6.

19.    The method according to claim 18 comprising the additional step of introducing into said
cloning vehicle an expression control sequence. according to claim 9, said expression control sequence
being introduced into said cloning vehicle so as to
control and to regulate the expression of said DNA
sequence.

20.    A method for producing a polypeptide
displaying an immunological or biological activity
of human interleukin 2, comprising the steps of transforming an appropriate host with a recombinant DNA
molecule according to any one of claims 8 to 10; culturing said host; and collecting said polypeptide.

21.    The method according to claim 20,
wherein the host is selected from the group consisting
of strains of E. coli, Pseudomonas, Bacillus subtilis,
Bacillus stearothermophilus, other bacilli, yeasts,
fungi, animal or plant hosts, and human tissue cells.

22.    A method for producing a polypeptide
displaying an immunological or biological activity of human interleukin 2 comprising the steps of culturing a host transformed by a recombinant DNA molecule according to any one of claims 8 to 10 and collecting said polypeptide.

23. A process for selecting a DNA sequence coding for a polypeptide displaying an immunological or biological activity of IL2 from a group of DNA sequences comprising the step of determining which of said DNA sequences hybridizes to a DNA sequence according to any one of claims 1 to 6.

24. A process for selecting a DNA sequence coding for a polypeptide displaying an immunological or biological activity of a non-human interleukin 2 comprising the step of determining which of said DNA sequences hybridize to a DNA sequence according to any one of claims 1 to 6.

25. The process of claim 23 or 24 wherein said DNA sequence screened is selected from the group consisting of DNA sequences from natural sources, synthetic DNA sequences, DNA sequences from recombinant DNA molecules and DNA sequences which are a combination of any of the foregoing DNA sequences.

26. A pharmaceutically acceptable composition for immunotherapy which comprises a pharmaceutically effective amount of at least one polypeptide selected from the group consisting of a polypeptide according to any one of claims 14 to 17.

27. A method of immunotherapy which comprises administering in a pharmaceutically acceptable manner an effective amount of a composition according to claim 26.

28. A composition for use as a proliferation agent in in vitro T-cell culture which comprises at least one polypeptide selected from the group consisting of a polypeptide according to any one of claims 14 to 17.

29. A method for proliferating in vitro T-cell cultures which comprises administering to the culture an effective amount of a composition according to claim 28.

30. An oligonucleotide derived or prepared from any one of the DNA sequences of claims 1 to 6 which selectively hybridizes to a DNA sequence encoding IL-2-like polypeptides.

31. A process for selecting a DNA sequence coding for an interleukin-related polypeptide comprising the step of determining which of said DNA sequences hybridizes to a DNA sequence according to any one of claims 1 to 6 or to an oligonucleotide of claim 30.

32. A pharmaceutically acceptable antiviral or anticancer composition which comprises a pharmaceutically effective amount of at least one polypeptide selected from the group consisting of a polypeptide according to any one of claims 14 to 17.

33. A method for antiviral or anticancer therapy which comprises administering in a pharmaceutically acceptable manner an effective amount of a composition according to claim 32.

Fig. 1

——— – GELATINE
- - - - - + GELATINE

Fig. 2

0118977

2/6

# Fig. 3

□ = 5' label
○ = 3' label

## Fig.4

AT CAC TCT CTT

0118977

4/6

```
                                    Met Tyr Arg Met Gln Leu Leu Ser Cys Ile Ala Leu Ser Leu Ala Leu Val Thr Asn
TAA·TCA CTA CTC ACA GTA ACC TCA·ACT·CCT GGC ACA ATG TAC AGG ATG·CAA CTC CTG TCT TGC ATT GCA CTA AGT CTT GCA·CTT GTC ACA AAC

Ser Ala Pro Thr Ser Ser Ser Thr Lys Lys Thr Gln Leu Gln Leu Glu His Leu Leu Leu Asp Leu Gln Met Ile Leu Asn Gly Ile Asn
AGT GCA CCT ACT TCA AGT TCT ACA AAG AAA ACA CAG CTA CAA CTG GAG CAT TTA CTG CTG GAT TTA CAG ATG ATT TTG AAT GGA ATT AAT

Asn Tyr Lys Asn Pro Lys Leu Thr Arg Met Leu Thr Phe Lys Phe Tyr Met Pro Lys Lys Ala Thr Glu Leu Lys His Leu Gln Cys Leu
AAT TAC AAG AAT CCC AAA CTC ACC AGG ATG CTC ACA TTT AAG TTT TAC ATG CCC AAG AAG GCC ACA GAA CTG AAA CAT CTT CAG TGT CTA

Glu Glu Glu Leu Lys Pro Leu Glu Glu Val Leu Asn Leu Ala Gln Ser Lys Asn Phe His Leu Arg Pro Arg Asp Leu Ile Ser Asn Ile
GAA GAA GAA CTC AAA CCT CTG GAG GAA GTG CTA AAT TTA GCT CAA AGC AAA AAC TTT CAC TTA AGA CCC AGG GAC TTA ATC AGC AAT ATC

Asn Val Ile Val Leu Glu Leu Lys Gly Ser Glu Thr Thr Phe Met Cys Glu Tyr Ala Asp Glu Thr Ala Thr Ile Val Glu Phe Leu Asn
AAC GTA ATA GTT CTG GAA CTA AAG GGA TCT GAA ACA ACA TTC ATG TGT GAA TAT GCT GAT GAG ACA GCA ACC ATT GTA GAA TTT CTG AAC

Arg Trp Ile Thr Phe Cys Gln Ser Ile Ile Ser Thr Leu Thr
AGA TGG ATT ACC TTT TGT CAA AGC ATC ATC TCA ACA CTG ACT|TGA|TAA TTA AGT GCT TCC CAC TTA AAA CAT ATC AGG CCT TCT ATT TAT

TTA AAT ATT TAA ATT TTA TAT TTA TTG TTG AAT GTA TGG TTT GCT ACC TAT TGT AAC TAT TAT TCT TAA TCT TAA AAC TAT AAA TAT GGA

TCT TTT ATG ATT CTT TTT GTA AGC CCT AGG GGC TCT AAA ATG GTT TCA CTT ATT TAT CCC AAA ATA TTT ATT ATT ATG TTG AAT GTT AAA

TAT AGT ATC TAT GTA GAT TGG TTA GTA AAA CTA TTT AAT AAA TTT GAT AA
```

Fig.5

Chromosomal DNA region encompassing the human interleukin 2 gene as present in λCH₄A-gHIL2-1

LEGEND : Arrows indicate the subcloned Eco RI restriction fragments.
: Symbols indicating restriction endonuclease cleavage sites (only sites present on the subcloned regions are indicated).

| Eco RI | Hind III | Bgl II | Xba I | Stu I |

: Asterisks stand for the presence of Eco RI synthetic linker sequences.
Above: open boxes and dashed lines indicate coding region areas and intervening sequences, respectively.

Fig.6

0118977

European Patent Office

**PARTIAL EUROPEAN SEARCH REPORT**
which under Rule 45 of the European Patent Convention
shall be considered, for the purposes of subsequent
proceedings, as the European search report

0118977
Application number

| DOCUMENTS CONSIDERED TO BE RELEVANT | | | EP 84300439.1 |
|---|---|---|---|
| Category | Citation of document with indication, where appropriate, of relevant passages | Relevant to claim | CLASSIFICATION OF THE APPLICATION (Int. Cl. 3) |
| X,P | EP - A1 - 0 091 539 (JAPANESE FOUNDATION FOR CANCER RESEARCH) <br><br> * Abstract; claims 19,21,24, 29; fig. 2 * <br><br> -- | 1-3,11- 13,17, 20 | C 12 N 15/00 <br> C 12 P 21/00 <br> C 07 C 103/52 <br> C 12 N 5/00 <br> A 61 K 37/02 <br> C 12 P 19/30 |
| A,P | EP - A2 - 0 088 195 (TAKEDA CHEMI- CAL INDUSTRIES, LTD.) <br><br> * Claim 9 * <br><br> ---- | 1,20 | |

TECHNICAL FIELDS
SEARCHED (Int. Cl. 3)

C 12 N
C 12 P
C 07 C 103/00
A 61 K

**INCOMPLETE SEARCH**

The Search Division considers that the present European patent application does not comply with the provisions of the European Patent Convention to such an extent that it is not possible to carry out a meaningful search into the state of the art on the basis of some of the claims.

Claims searched completely: 27
Claims searched incompletely: —
Claims not searched: 33
Reason for the limitation of the search:

(Art. 52(4) EPC)

| Place of search | Date of completion of the search | Examiner |
|---|---|---|
| VIENNA | 16-05-1984 | WOLF |